(19) Europäisches Patentamt
European Patent Office
Office européen des brevets

(11) Veröffentlichungsnummer: **0 371 240 B1**

(12) # EUROPÄISCHE PATENTSCHRIFT

(45) Veröffentlichungstag der Patentschrift: **16.06.93**

(51) Int. Cl.$^5$: **C07D 265/10**, A01N 43/76

(21) Anmeldenummer: **89119135.5**

(22) Anmeldetag: **14.10.89**

Die Akte enthält technische Angaben, die nach dem Eingang der Anmeldung eingereicht wurden und die nicht in dieser Patentschrift enthalten sind.

(54) **Verwendung von N-Phenyl-substituierten Oxazindionen als Herbizide sowie N-Phenyl-substituierte Oxazindione und mehrere Verfahren zu deren Herstellung.**

(30) Priorität: **28.10.88 DE 3836742**

(43) Veröffentlichungstag der Anmeldung:
**06.06.90 Patentblatt 90/23**

(45) Bekanntmachung des Hinweises auf die Patenterteilung:
**16.06.93 Patentblatt 93/24**

(84) Benannte Vertragsstaaten:
**BE CH DE FR GB IT LI NL**

(56) Entgegenhaltungen:
**DE-A- 2 132 763**

**CHEMICAL ABSTRACTS, Band 89, Nr. 17, 23. Oktober 1978, Columbus, Ohio, USA; CAPUANO L. et al.: "Syntheses with 2-diazo-1,3-diketones. III. Cyclocondensations with H-nucleophiles", Seite 608, Spalte 1, Zusammenfassung-Nr. 146867x**

**CHEMICAL ABSTRACTS, Band 87, Nr. 21, 21. November 1977, Columbus, Ohio, USA; NIPPON SODA CO.: "Oxazine derivatives", Seite 580, Spalte 1, Zusammenfassung-Nr. 168049t**

(73) Patentinhaber: **BAYER AG**

**W-5090 Leverkusen 1 Bayerwerk(DE)**

(72) Erfinder: **Ooms, Pieter, Dr.**
**Doerperhofstrasse 16**
**W-4150 Krefeld 1(DE)**
Erfinder: **Kunisch, Franz, Dr.**
**Zum Hahnenberg 20**
**W-5068 Odenthal(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Strang, Harry, Dr.**
**Heiderweg 53**
**W-4000 Düsseldorf 31(DE)**

**EP 0 371 240 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 74, Nr. 11, 15. März 1971, Columbus, Ohio, USA; MITSUI TOATSU CHEMICALS CO.: "3,4-dihydro-2,4-dioxo-6-methyl-2H-1,3-oxazines and 3-carbamyl-2,6-dimethyl-4-pyrones", Seite 370, Spalte 2, Zusammenfassung-Nr. 53811z

## Beschreibung

Die Erfindung betrifft die Verwendung von neuen und bekannten N-Phenyl-substituierten Oxazindionen als Herbizide sowie neue N-Phenyl-substituierte Oxazindione und mehrere Verfahren zu ihrer Herstellung.

Es ist bekannt, daß bestimmte heterocyclische Verbindungen, wie z. B. 4-Amino-3-methyl-6-phenyl-4H-1,2,4-triazin-5-on (Metamitron, GOLTIX) herbizid wirksam sind (vgl. DE-OS 23 64 474). Die Wirkung dieser bekannten Verbindung ist jedoch nicht in allen Belangen zufriedenstellend.

Weiter sind eine Reihe von substituierten Oxazindionen, jedoch nicht ihre herbiziden Eigenschaften, aus der Literatur bekannt (vgl. Chem. Ber. 109 (1976), 2456 - 2461; Tetrahedron Lett. 1967, 2089 - 2092; Liebigs Ann. Chem. 1976, 1689 - 1712; J. Heterocycl. Chem. 18 (1981), 1095 - 1100; J. Org. Chem. 49 (1984), 5105 - 5108; Monatsh. Chem. 94 (1963), 544 - 548; DE-OS 21 32 763; DE-AS 22 07 549; DE-OS 20 05 118).

Zunächst werden in der vorliegenden Anmeldung die neuen N-Phenyl-substituierten Oxazindione der Formel (I) und anschließend die neuen und bekannten N-Phenyl-substituierten Oxazindione der Formel (IA) beschrieben.

Es wurden nun neue substituierte Oxazindione der allgemeinen Formel (I)

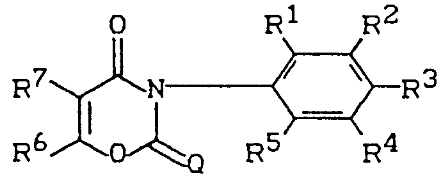

(I)

in welcher

- $R^1$ für Wasserstoff oder Halogen steht,
- $R^2$ für Wasserstoff, Nitro, Cyano, Halogen steht oder für einen gegebenenfalls durch Fluor, Chlor, Brom, Cyano, $C_3$-$C_6$-Cycloalkyl, welches gegebenenfalls durch Fluor, Chlor, Brom, Methyl und/oder Ethyl substituiert ist, durch $C_1$-$C_6$-Alkoxy, $C_1$-$C_4$-Alkoxy-$C_1$-$C_4$-alkoxy, $C_1$-$C_6$-Alkylthio, $C_1$-$C_6$-Alkylsulfinyl, $C_1$-$C_6$-Alkylsulfonyl, $C_1$-$C_4$-Alkylthio-$C_1$-$C_4$-alkoxy, $C_1$-$C_4$-Alkylsulfonyl-$C_1$-$C_4$-alkoxy, $C_3$-$C_6$-Alkenyloxy, $C_3$-$C_6$-Alkinyloxy, $C_3$-$C_6$-Alkenylthio, $C_3$-$C_6$-Alkinylthio, Carboxy, $C_1$-$C_6$-Alkoxy-carbonyl, $C_3$-$C_6$-Alkenyloxy-carbonyl, $C_3$-$C_6$-Alkinyloxy-carbonyl, $C_3$-$C_6$-Cycloalkyloxy-carbonyl, $C_3$-$C_6$-Cycloalkenyloxy-carbonyl, $C_3$-$C_6$-Cycloalkyl-$C_1$-$C_4$-alkoxy-carbonyl, $C_3$-$C_6$-Cycloalkenyl-$C_1$-$C_4$-alkoxy-carbonyl, $C_1$-$C_4$-Alkoxy-$C_1$-$C_4$-alkoxy-carbonyl, $C_1$-$C_4$-Alkylthio-$C_1$-$C_4$-alkoxy-carbonyl, Benzyloxy-$C_1$-$C_4$-alkoxy-carbonyl, $C_1$-$C_4$-Alkoxy-carbonyl-$C_1$-$C_4$-alkoxy-carbonyl, $C_1$-$C_4$-Alkylamino-carbonyl-$C_1$-$C_4$-alkoxy-carbonyl, Di-($C_1$-$C_4$-alkyl)-amino-carbonyl-$C_1$-$C_4$-alkoxy-carbonyl, $C_1$-$C_4$-Alkoxy-$C_1$-$C_4$-alkyl-amino-carbonyl, $C_1$-$C_4$-Alkoxy-imino-$C_1$-$C_4$-alkyl, $C_3$-$C_4$-Alkenyloxy-imino-$C_1$-$C_4$-alkyl, $C_1$-$C_4$-Alkoxy-carbonyl-$C_1$-$C_4$-alkoxy-imino-$C_1$-$C_4$-alkyl, $C_1$-$C_6$-Alkylthio-carbonyl, $C_3$-$C_6$-Alkenylthio-carbonyl, $C_3$-$C_6$-Alkinylthio-carbonyl, Carbamoyl, $C_1$-$C_6$-Alkylamino-carbonyl, $C_3$-$C_6$-Alkenylamino-carbonyl, $C_3$-$C_6$-Alkinylamino-carbonyl, Di-($C_1$-$C_4$-alkyl)-amino-carbonyl, Di-($C_3$-$C_4$-alkenyl)-amino-carbonyl und/oder Di-($C_3$-$C_4$-alkinyl)-amino-carbonyl substituierten Rest aus der Reihe $C_1$-$C_8$-Alkyl, $C_1$-$C_6$-Alkoxy, $C_1$-$C_6$-Alkylthio und $C_1$-$C_6$-Alkylamino oder für einen gegebenenfalls durch Fluor und/oder Chlor substituierten Rest aus der Reihe $C_3$-$C_6$-Alkenyl, $C_3$-$C_6$-Alkinyl, $C_3$-$C_6$-Alkenyloxy, $C_3$-$C_6$-Alkinyloxy, $C_3$-$C_6$-Alkenylthio, $C_3$-$C_6$-Alkinylthio, $C_1$-$C_6$-Alkylsulfinyl und $C_1$-$C_6$-Alkylsulfonyl steht,
- $R^3$ für Wasserstoff, Halogen, Nitro, Cyano oder für einen gegebenenfalls durch Fluor und/oder Chlor substituierten Rest aus der Reihe $C_1$-$C_4$-Alkyl, $C_3$-$C_4$-Alkenyl, $C_3$-$C_4$-Alkinyl, $C_1$-$C_4$-Alkoxy, $C_3$-$C_4$-Alkenyloxy, $C_3$-$C_4$-Alkinyloxy, $C_1$-$C_4$-Alkylthio, $C_3$-$C_4$-Alkenylthio, $C_3$-$C_4$-Alkinylthio, $C_1$-$C_4$-Alkylsulfinyl, $C_1$-$C_4$-Alkylsulfonyl und $C_1$-$C_4$-Alkylamino steht, oder die beiden Reste
- $R^2$ und $R^3$ zusammen für $-O-CH_2-O-CH_2-$ oder die Gruppierung $-X-(CO)_n-A-Y-$ stehen, worin
- n für die Zahlen 0 oder 1 steht,
- A für eine direkte Bindung oder für geradkettiges oder verzweigtes und gegebenenfalls durch Fluor und/oder Chlor substituiertes $C_1$-$C_4$-Alkandiyl steht,
- X für Sauerstoff, Schwefel oder die Gruppierung $N-R^8$ steht worin,
- $R^8$ für Wasserstoff oder gegebenenfalls durch Fluor und/oder Chlor, Cyano, $C_1$-$C_4$-Alkoxy,

$C_1$-$C_4$-Alkoxy-carbonyl oder Pyridyl substituierte Reste aus der Reihe $C_1$-$C_4$-Alkyl, $C_3$-$C_4$-Alkenyl und $C_3$-$C_4$-Alkinyl steht und

Y für Sauerstoff oder Schwefel steht,

in welcher weiter

$R^4$ für Wasserstoff oder Halogen steht,

$R^5$ für Wasserstoff oder Halogen steht,

$R^6$ für Wasserstoff, Halogen oder gegebenenfalls durch Fluor und/oder Chlor substituiertes $C_1$-$C_4$-Alkyl steht,

$R^7$ für Wasserstoff, Halogen oder gegebenenfalls durch Fluor und/oder Chlor substituiertes $C_1$-$C_4$-Alkyl steht oder die beiden Reste

$R^6$ und $R^7$ zusammen für geradkettiges oder verzweigtes $C_3$-$C_5$-Alkandiyl stehen oder zusammen mit den Kohlenstoffatomen, an die sie gebunden sind, eine Benzogruppierung bilden, und

Q für Sauerstoff oder Schwefel steht,

mit der Maßgabe, daß wenigstens drei der Reste $R^1$ bis $R^5$ von Wasserstoff verschieden sind oder die beiden Reste $R^2$ und $R^3$ zusammen für $-O-CH_2-O-CH_2-$ oder die Gruppierung $-X-(CO)_n-A-Y-$ stehen, gefunden.

Man erhält die neuen N-Phenyl-substituierten Oxazindione der allgemeinen Formel (I), wenn man Aryliso(thio)cyanate der allgemeinen Formel (II)

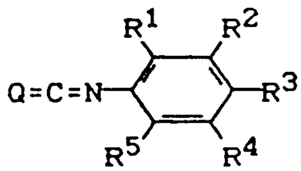

(II)

in welcher

Q, $R^1$, $R^2$, $R^3$, $R^4$ und $R^5$ die oben angegebenen Bedeutungen haben,

(a) mit 1,3-Dioxin-4-onen der allgemeinen Formel (III)

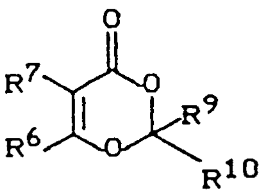

(III)

in welcher

$R^6$ und $R^7$ die oben angegebenen Bedeutungen haben und

$R^9$ und $R^{10}$ für Wasserstoff oder gegebenenfalls substituiertes Alkyl stehen,

gegebenenfalls in Gegenwart eines Verdünnungsmittels umsetzt, oder

(b) mit Dicarbonsäuredichloriden der allgemeinen Formel (IV)

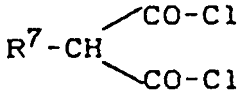

(IV)

in welcher

$R^7$ die oben angegebene Bedeutung hat,

gegebenenfalls in Gegenwart eines Verdünnungsmittels umsetzt, oder

(c) mit 2-Diazo-1,3-diketonen der allgemeinen Formel (V)

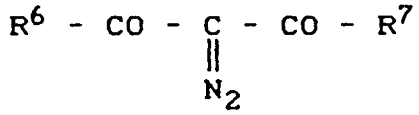

(V)

in welcher

$R^6$ und $R^7$ die oben angegebenen Bedeutungen haben,

gegebenenfalls in Gegenwart eines Verdünnungsmittels umsetzt, oder

(d) mit Salicylsäure oder deren Estern der allgemeinen Formel (VI)

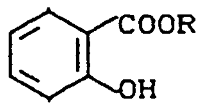

(VI)

in welcher

R für Wasserstoff oder Alkyl steht,

gegebenenfalls in Gegenwart eines Säureakzeptors und gegebenenfalls in Gegenwart eines Verdünnungsmittels umsetzt.

Weitere Synthesewege zu den substituierten Oxazindionen sind nachstehend skizziert, wobei $R^1$ bis $R^{10}$ und Q die oben angegebenen Bedeutungen haben:

(e) Umsetzung von Aryliso(thio)cyanaten (II) mit 5-Acyl-1,3-dioxan-4,6-dionen (VII):

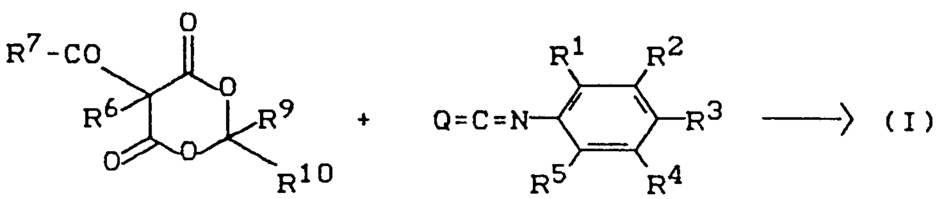

(VII) (II)

(f) Umsetzung von Aryliso(thio)cyanaten (II) mit Diketenen (VIII):

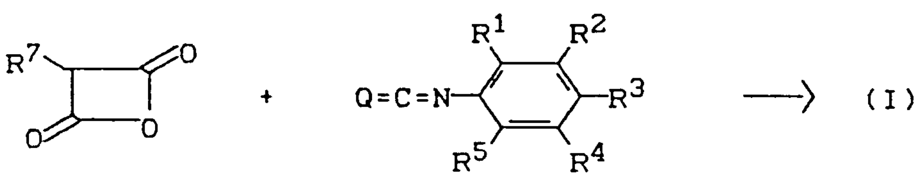

(VIII) (II)

(g) Umsetzung von Aryliso(thio)cyanaten (II) mit Ethoxyalkinonen (IX) - (R: $C_2H_5$):

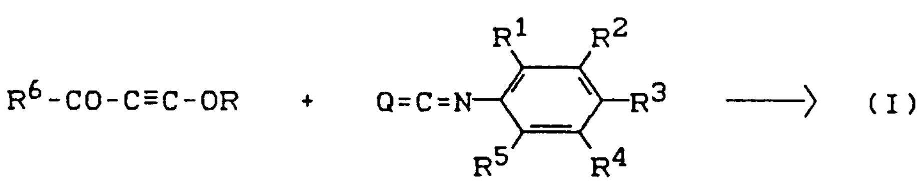

(IX) (II)

(h) Umsetzung von Oxazindionen (Ia) mit Halogenen ($X_2$: Chlor oder Brom):

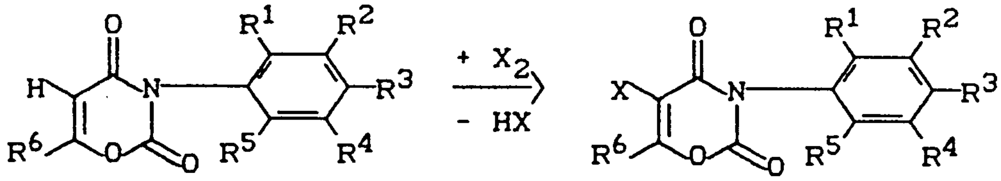


(Ia) (Ib)

Die neuen substituierten Oxazindione der allgemeinen Formel (I) zeichnen sich durch starke herbizide Wirksamkeit aus. Auch die oben durch Disclaimer ausgenommenen bekannten Verbindungen der Formel (I) zeigen sehr gute herbizide Wirksamkeit.

In den Definitionen sind die gesättigten oder ungesättigten Kohlenwasserstoffketten wie beispielsweise Alkyl, Alkoxy, Alkylsulfonyl, Alkenyl, Alkinyl, Alkenyloxy oder Cycloalkylalkyl, jeweils geradkettig oder verzweigt.

Die Verknüpfung in der Aufzählung der Reste mit "und/oder" bedeutet, daß die Substitution einfach oder mehrfach, gleich oder verschieden erfolgen kann.

Halogen steht im allgemeinen für Fluor, Chlor, Brom oder Iod. Vorzugsweise steht Halogen für Fluor, Chlor oder Brom.

Die Erfindung betrifft nun die Verwendung der erfindungsgemäßen neuen Verbindungen der Formel (I) und der bekannten substituierten Oxazindione, welche in der nachstehenden Formel (IA) zusammengefaßt sind,

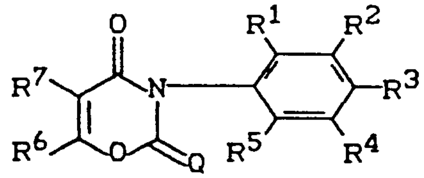

(IA)

in welcher

$R^1$, $R^2$, $R^3$, $R^4$, $R^5$, $R^6$, $R^7$ und Q die oben angegebenen Bedeutungen haben, jedoch ohne die für die erfindungsgemäßen Verbindungen der Formel (I) für $R^1$, $R^2$, $R^3$, $R^4$, $R^5$ angegebenen Beschränkungen.

Überraschenderweise sind die erfindungsgemäßen substituierten Oxazindione der allgemeinen Formel (I) bzw. (IA) gegen wichtige Unkräuter erheblich stärker wirksam als beispielsweise 4-Amino-3-methyl-6-phenyl-4H-1,2,4-triazin-5-on, welches ein strukturell ähnlicher vorbekannter Wirkstoff gleicher Wirkungsrichtung ist.

Die Erfindung betrifft insbesondere Verbindungen der allgemeinen Formel (I), in welcher

$R^1$ für Wasserstoff steht,

$R^2$ für Wasserstoff, Nitro, Cyano, Fluor, Chlor oder Brom oder für einen gegebenenfalls durch Fluor, Chlor, Cyclopropyl, welches durch Chlor und/oder Methyl substituiert sein kann; durch $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, Carboxy, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl, Di-($C_1$-$C_2$-alkyl)-aminocarbonyl, $C_1$-$C_2$-Alkoxy-$C_1$-$C_2$-alkyl-amino-carbonyl, $C_3$-$C_4$-Alkenyloxy-imino-$C_1$-$C_2$-alkyl, $C_1$-$C_2$-Alkoxy-imino-$C_1$-$C_2$-alkyl, $C_1$-$C_2$-Alkoxy-carbonyl-$C_1$-$C_2$-alkoxy-imino-$C_1$-$C_2$-alkyl substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Cyano, Methyl oder Trifluormethyl steht, oder die beiden Reste

| | |
|---|---|
| $R^2$ und $R^3$ | zusammen für $-O-CH_2-O-CH_2-$ oder die Gruppierung $-X-(CO)_n-A-Y-$ stehen, worin |
| n | für die Zahlen 0 oder 1 steht, |
| A | für eine direkte Bindung oder für Methylen, Ethylen, Propylen oder Butylen steht, |
| X | für Sauerstoff oder die Gruppierung $N-R^8$ steht, worin |
| $R^8$ | für Wasserstoff, $C_1-C_4$-Alkyl, $C_3-C_4$-Alkenyl oder $C_3-C_4$-Alkinyl steht und |
| Y | für Sauerstoff steht, |

in welcher weiter

| | |
|---|---|
| $R^4$ | für Wasserstoff steht, |
| $R^5$ | für Wasserstoff, Fluor oder Chlor steht |
| $R^6$ | für Wasserstoff, Chlor, Brom, Methyl, Ethyl, Propyl, Isopropyl oder Trifluormethyl steht, |
| $R^7$ | für Wasserstoff, Chlor, Brom, Methyl, Ethyl, Propyl, Isopropyl oder Trifluormethyl steht oder die beiden Reste |
| $R^6$ und $R^7$ | zusammen für geradkettiges oder verzweigtes $C_3-C_5$-Alkandiyl stehen oder zusammen mit den Kohlenstoffatomen, an die sie gebunden sind, eine Benzogruppierung bilden, und |
| Q | für Sauerstoff oder Schwefel steht, |

- mit der Maßgabe, daß die Reste $R^2$, $R^3$ und $R^5$ von Wasserstoff verschieden sind oder die beiden Reste $R^2$ und $R^3$ zusammen für $-O-CH_2-O-CH_2-$ oder die Gruppierung $X-(CO)_n-A-Y-$ stehen, worin n, A, X und Y die oben als besonders bevorzugt angegebenen Bedeutungen haben.

$R^1$, $R^2$, $R^3$, $R^4$, $R^5$, $R^6$, $R^7$ und Q in der Formel (IA) besitzen vorzugsweise bzw. insbesondere diejenigen Bedeutungen, die bei der Beschreibung der Verbindungen der Formel (I) als bevorzugt bzw. als besonders bevorzugt für $R^1$, $R^2$, $R^3$, $R^4$, $R^5$, $R^6$, $R^7$ und Q angegeben wurden.

Beispiele für die erfindungsgemäßen Verbindungen der Formel (I) wie auch der Formel (IA) sind in der nachstehenden Tabelle 1 aufgeführt.

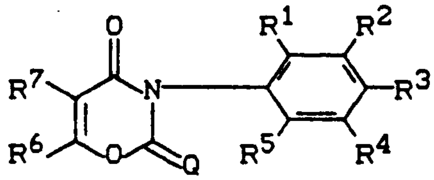

(I/IA)

<u>Tabelle 1</u>: Beispiele für die erfindungsgemäßen Verbindungen der Formel (I) bzw. (IA)

| $R^1$ | $R^2$ | $R^3$ | $R^4$ | $R^5$ | $R^6$ | $R^7$ | Q |
|---|---|---|---|---|---|---|---|
| H | $OCH_3$ | Cl | H | F | $CH_3$ | H | O |
| H | $OCH_3$ | Cl | H | Cl | $CH_3$ | H | O |
| H | $C_2H_5$ | Cl | H | Cl | $CH_3$ | H | O |
| H | $OC_2H_5$ | Cl | H | F | $CH_3$ | H | O |
| H | $OCH(CH_3)_2$ | Cl | H | F | $CH_3$ | H | O |
| H | $OCH(CH_3)_2$ | Cl | H | Cl | $CH_3$ | H | O |
| H | $OC_4H_9$ | Cl | H | Cl | $CH_3$ | H | O |
| H | $OC_4H_9$ | Cl | H | F | $CH_3$ | H | O |
| H | $OCHF_2$ | Cl | H | F | $CH_3$ | H | O |
| H | $OCHF_2$ | Cl | H | Cl | $CH_3$ | H | O |
| H | $OCH_3$ | Cl | H | Cl | $CH_3$ | H | S |
| H | $OCH_3$ | Cl | H | F | $CH_3$ | H | S |
| H | $CH_3$ | Cl | H | F | $CH_3$ | H | O |
| H | $C_2H_5$ | Cl | H | F | $CH_3$ | H | O |
| H | $CH_3$ | Cl | H | Cl | $CH_3$ | H | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $C_2H_5$ | Cl | H | F | $CH_3$ | H | O |
| H | $SCH_3$ | Cl | H | F | $CH_3$ | H | O |
| H | $SCH_3$ | Cl | H | Cl | $CH_3$ | H | O |
| H | $SC_2H_5$ | Cl | H | Cl | $CH_3$ | H | O |
| H | $SC_2H_5$ | Cl | H | F | $CH_3$ | H | O |
| H | $OCH_2COOCH_3$ | Cl | H | Cl | $CH_3$ | H | O |
| H | $OCH_2COOCH_3$ | Cl | H | F | $CH_3$ | H | O |
| H | $OCH_2COOC_2H_5$ | Cl | H | F | $CH_3$ | H | O |
| H | $OCH_2COOC_2H_5$ | Cl | H | Cl | $CH_3$ | H | O |
| H | $OCHCOOCH_3$<br>\|<br>$CH_3$ | Cl | H | Cl | $CH_3$ | H | O |
| H | $OCHCOOCH_3$<br>\|<br>$CH_3$ | Cl | H | F | $CH_3$ | H | O |
| H | $OCHCOOC_2H_5$<br>\|<br>$CH_3$ | Cl | H | F | $CH_3$ | H | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $OCHCOOC_2H_5$<br>\|<br>$CH_3$ | Cl | H | Cl | $CH_3$ | H | O |
| H | $OCH_2CH{=}CH_2$ | Cl | H | Cl | $CH_3$ | H | O |
| H | $OCH_2CH{=}CH_2$ | Cl | H | F | $CH_3$ | H | O |
| H | $OCH_2C{\equiv}CH$ | Cl | H | F | $CH_3$ | H | O |
| H | $OCH_2C{\equiv}CH$ | Cl | H | Cl | $CH_3$ | H | O |
| H | $-NH-CO-CH_2-O-$ | | H | H | $CH_3$ | H | O |
| H | $-NH-CO-CH_2-O-$ | | H | F | $CH_3$ | H | O |
| H | $-NH-CO-CH_2-O-$ | | H | Cl | $CH_3$ | H | O |
| H | Cl | F | Cl | F | $CH_3$ | H | O |
| H | $OCH(CH_3)_2$ | Br | H | F | $CH_3$ | H | O |
| H | $OCH_3$ | Br | H | F | $CH_3$ | H | O |
| H | $OC_3H_7$ | Cl | H | F | $CH_3$ | H | O |
| H | $OCH_2CH(CH_3)_2$ | Cl | H | F | $CH_3$ | H | O |
| H | $CH_2COOCH_3$ | Cl | H | F | $CH_3$ | H | S |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $OCH_2CH=CH_2$ | Cl | H | F | $CH_3$ | H | S |
| H | $OCH_2C≡CH$ | Cl | H | F | $CH_3$ | H | S |
| H | $OCH_2COOCH_3$ | Cl | H | Cl | $CH_3$ | H | S |
| H | $OCH_2CH=CH_2$ | Cl | H | Cl | $CH_3$ | H | S |
| H | $OCH_2C≡CH$ | Cl | H | Cl | $CH_3$ | H | S |
| H | $OCHCH_2CH_3$<br>\|<br>$CH_3$ | Cl | H | F | $CH_3$ | H | O |
| H | $OCH_2COOCH(CH_3)_2$ | Cl | H | F | $CH_3$ | H | O |
| H | $OCH_2COO$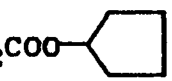 | Cl | H | F | $CH_3$ | H | O |
| H | $OCH_2COOC_5H_{11}$ | Cl | H | F | $CH_3$ | H | O |
| H | $OCH_2COOCH_2$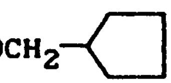 | Cl | H | F | $CH_3$ | H | O |
| H | $OCH_2CH_2OCH_3$ | Cl | H | F | $CH_3$ | H | O |
| H | $OCH_2CH_2OC_2H_5$ | Cl | H | F | $CH_3$ | H | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $OCH_2CH_2SCH_3$ | Cl | H | F | $CH_3$ | H | O |
| H | $OCH_2CH_2SC_2H_5$ | Cl | H | F | $CH_3$ | H | O |
| H | $SCH_2COOCH_3$ | Cl | H | F | $CH_3$ | H | O |
| H | $SCH_2COOC_2H_5$ | Cl | H | F | $CH_3$ | H | O |
| H | $SCH_2COOC_5H_{11}$ | Cl | H | F | $CH_3$ | H | O |
| H | $SCH_2COO$ 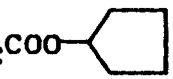 | Cl | H | F | $CH_3$ | H | O |
| H | $SCH_2COOCH_2$ 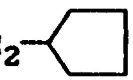 | Cl | H | F | $CH_3$ | H | O |
| H | $SCH(CH_3)_2$ | Cl | H | F | $CH_3$ | H | O |
| H | $SC_4H_9$ | Cl | H | F | $CH_3$ | H | O |
| H | $SCH_2CH=CH_2$ | Cl | H | F | $CH_3$ | H | O |
| H | $SCH_2C\equiv CH$ | Cl | H | F | $CH_3$ | H | O |
| H | $OCH_2COOCH_2CH=CH_2$ | Cl | H | F | $CH_3$ | H | O |
| H | $OCH_2COOCH_2C\equiv CH$ | Cl | H | F | $CH_3$ | H | O |

**Tabelle 1 - Fortsetzung**

| $R^1$ | $R^2$ | $R^3$ | $R^4$ | $R^5$ | $R^6$ | $R^7$ | Q |
|---|---|---|---|---|---|---|---|
| H | $OCH_2CON(CH_3)_2$ | Cl | H | F | $CH_3$ | H | O |
| H | 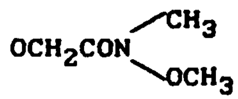 | Cl | H | F | $CH_3$ | H | O |
| H | 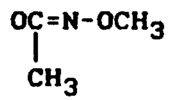 | Cl | H | F | $CH_3$ | H | O |
| H | $OCH_2CH=N-OCH_3$ | Cl | H | F | $CH_3$ | H | O |
| H | 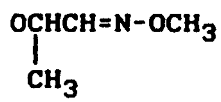 | Cl | H | F | $CH_3$ | H | O |
| H | $OCH_2CH=N-OCH_2CH_2=CH_2$ | Cl | H | F | $CH_3$ | H | O |
| H | $OCH_2CH=N-OCH_2COOCH_3$ | Cl | H | F | $CH_3$ | H | O |
| H | $SCH_2COOCH_2CH=CH_2$ | Cl | H | F | $CH_3$ | H | O |
| H | $SCH_2COOCH_2C{\equiv}CH$ | Cl | H | F | $CH_3$ | H | O |
| H | $OCH_2COOCH_2COOCH_3$ | Cl | H | F | $CH_3$ | H | O |
| H | 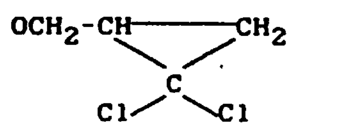 | Cl | H | F | $CH_3$ | H | O |

<u>Tabelle 1</u> - Fortsetzung

| $R^1$ | $R^2$ | $R^3$ | $R^4$ | $R^5$ | $R^6$ | $R^7$ | Q |
|---|---|---|---|---|---|---|---|
| H | 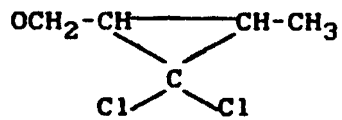 | Cl | H | F | $CH_3$ | H | O |
| H | $OCHF_2$ | Cl | H | F | $CH_3$ | H | S |
| H | $OCF_2CHFCl$ | Cl | H | Cl | $CH_3$ | H | O |
| H | $OCF_2CHFCl$ | Cl | H | F | $CH_3$ | H | O |
| H | $OCF_2CHF_2$ | Cl | H | F | $CH_3$ | H | O |
| H | $OCH_2CH=CHCl$ | Cl | H | F | $CH_3$ | H | O |
| H | $OCF_2Cl$ | Cl | H | F | $CH_3$ | H | O |
| H | $OCF_3$ | Cl | H | F | $CH_3$ | H | O |
| H | 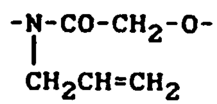 | | H | H | $CH_3$ | H | O |
| H | 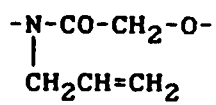 | | H | F | $CH_3$ | H | O |
| H | 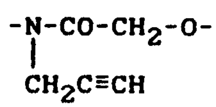 | | H | H | $CH_3$ | H | O |

**Tabelle 1 - Fortsetzung**

| $R^1$ | $R^2$ | $R^3$ | $R^4$ | $R^5$ | $R^6$ | $R^7$ | Q |
|---|---|---|---|---|---|---|---|
| H | $-N(CH_2C\equiv CH)-CO-CH_2-O-$ | | H | F | $CH_3$ | H | O |
| H | $OCH_2COOC_5H_{11}$ | $CH_3$ | H | F | $CH_3$ | H | O |
| H | $OCH_2=CH_2$ | $CH_3$ | H | F | $CH_3$ | H | O |
| H | $OCH_2C\equiv CH$ | $CH_3$ | H | F | $CH_3$ | H | O |
| H | $OCH_2COOCH_2C\equiv CH$ | $CH_3$ | H | F | $CH_3$ | H | O |
| H | $OCH_2COO$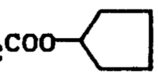 | $CH_3$ | H | F | $CH_3$ | H | O |
| H | $SCH_2COO$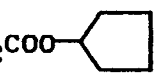 | $CH_3$ | H | F | $CH_3$ | H | O |
| H | $SCH_2COOCH_2C\equiv CH$ | $CH_3$ | H | F | $CH_3$ | H | O |
| H | $(OCH_2CH_2)_2OC_2H_5$ | Br | H | F | $CH_3$ | H | S |
| H | $(OCH_2CH_2)_2OC_2H_5$ | Cl | H | F | $CH_3$ | H | O |
| H | $(OCH_2CH_2)_2OCH_3$ | Cl | H | F | $CH_3$ | H | O |
| H | $OCH_3$ | Cl | H | Cl | $-(CH_2)_3-$ | | O |

**Tabelle 1 - Fortsetzung**

| $R^1$ | $R^2$ | $R^3$ | $R^4$ | $R^5$ | $R^6$ $R^7$ | Q |
|---|---|---|---|---|---|---|
| H | $OCH_3$ | Cl | H | F | $-(CH_2)_3-$ | O |
| H | $OCH_3$ | Cl | H | F | $-(CH_2)_4-$ | O |
| H | $OCH_3$ | Cl | H | Cl | $-(CH_2)_4-$ | O |
| H | $OC_2H_5$ | Cl | H | F | $-(CH_2)_3-$ | O |
| H | $OC_2H_5$ | Cl | H | F | $-(CH_2)_4-$ | O |
| H | $OC_3H_7$ | Cl | H | F | $-(CH_2)_3-$ | O |
| H | $OCH(CH_3)_2$ | Cl | H | F | $-(CH_2)_3-$ | O |
| H | $OC_4H_9$ | Cl | H | F | $-(CH_2)_3-$ | O |
| H | $OCH_2CH(CH_3)_2$ | Cl | H | F | $-(CH_2)_3-$ | O |
| H | $OCH_2CH=CH_2$ | Cl | H | F | $-(CH_2)_3-$ | O |
| H | $OCH_2C{\equiv}CH$ | Cl | H | F | $-(CH_2)_3-$ | O |
| H | $OCH_2COOCH_3$ | Cl | H | F | $-(CH_2)_3-$ | O |
| H | $OCH_3$ | Cl | H | F | $-(CH_2)_3-$ | S |
| H | $OCH(CH_3)_2$ | Cl | H | F | $-(CH_2)_3-$ | S |
| H | $OCH_2COOCH_3$ | Cl | H | F | $-(CH_2)_3-$ | S |

**Tabelle 1 - Fortsetzung**

| $R^1$ | $R^2$ | $R^3$ | $R^4$ | $R^5$ | $R^6$ $R^7$ | Q |
|---|---|---|---|---|---|---|
| H | $OCHF_2$ | Cl | H | F | $-(CH_2)_3-$ | S |
| H | $OCHF_2$ | Cl | H | F | $-(CH_2)_3-$ | O |
| H | $OCHF_2$ | Cl | H | F | $-(CH_2)_4-$ | O |
| H | $CH_3$ | Cl | H | F | $-(CH_2)_3-$ | O |
| H | $C_2H_5$ | Cl | H | F | $-(CH_2)_3-$ | O |
| H | $SCH_3$ | Cl | H | Cl | $-(CH_2)_3-$ | O |
| H | $SCH_3$ | Cl | H | F | $-(CH_2)_3-$ | O |
| H | $SC_2H_5$ | Cl | H | F | $-(CH_2)_3-$ | O |
| H | $SC_2H_5$ | Cl | H | F | $-(CH_2)_4-$ | O |
| H | $OCH_2COOCH_3$ | Cl | H | F | $-(CH_2)_4-$ | O |
| H | $OCH_2COOC_2H_5$ | Cl | H | F | $-(CH_2)_3-$ | O |
| H | $OCH(CH_3)COOCH_3$ | Cl | H | F | $-(CH_2)_3-$ | O |
| H | $OCH(CH_3)COOC_2H_5$ | Cl | H | F | $-(CH_2)_3-$ | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $-NH-CO-CH_2-O-$ | | H | H | $-(CH_2)_3-$ | | O |
| H | $-NH-CO-CH_2-O-$ | | H | F | $-(CH_2)_3-$ | | O |
| H | $-NH-CO-CH_2-O-$ | | H | Cl | $-(CH_2)_3-$ | | O |
| H | $OCH_3$ | Br | H | F | $-(CH_2)_3-$ | | O |
| H | $OCH_2COO$ 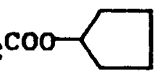 | Cl | H | F | $-(CH_2)_3-$ | | O |
| H | $OCH_2COOC_5H_{11}$ | Cl | H | F | $-(CH_2)_3-$ | | O |
| H | $OCH_2COOCH_2$ 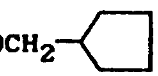 | Cl | H | F | $-(CH_2)_3-$ | | O |
| H | $OCH_2CH_2OCH_3$ | Cl | H | F | $-(CH_2)_3-$ | | O |
| H | $OCH_2CH_2OC_2H_5$ | Cl | H | F | $-(CH_2)_3-$ | | O |
| H | $OCH_2CH_2SCH_3$ | Cl | H | F | $-(CH_2)_3-$ | | O |
| H | $OCH_2CH_2SC_2H_5$ | Cl | H | F | $-(CH_2)_3-$ | | O |
| H | $SCH_2COOCH_3$ | Cl | H | F | $-(CH_2)_3-$ | | O |
| H | $SCH_2COOC_2H_5$ | Cl | H | F | $-(CH_2)_3-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $SCH_2COOCH(CH_3)_2$ | Cl | H | F | $-(CH_2)_3-$ | | O |
| H | $SCH_2COOC_4H_9$ | Cl | H | F | $-(CH_2)_3-$ | | O |
| H | $SCH_2COOC_5H_{11}$ | Cl | H | F | $-(CH_2)_3-$ | | O |
| H | $SCH_2COO$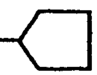 | Cl | H | F | $-(CH_2)_3-$ | | O |
| H | $SCH_2COOCH_2$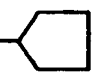 | Cl | H | F | $-(CH_2)_3-$ | | O |
| H | $SCH(CH_3)_2$ | Cl | H | F | $-(CH_2)_3-$ | | O |
| H | $SC_4H_9$ | Cl | H | F | $-(CH_2)_3-$ | | O |
| H | $SCH_2CH=CH_2$ | Cl | H | F | $-(CH_2)_3-$ | | O |
| H | $SCH_2C\equiv CH$ | Cl | H | F | $-(CH_2)_3-$ | | O |
| H | $OCH_2COOCH_2CH=CH_2$ | Cl | H | F | $-(CH_2)_3-$ | | O |
| H | $OCH_2COOCH_2C\equiv CH$ | Cl | H | F | $-(CH_2)_3-$ | | O |
| H | $SCH_2COOCH_2CH=CH_2$ | Cl | H | F | $-(CH_2)_3-$ | | O |
| H | $SCH_2COOCH_2C\equiv CH$ | Cl | H | F | $-(CH_2)_3-$ | | O |

<u>Tabelle 1</u> - Fortsetzung

| $R^1$ | $R^2$ | $R^3$ | $R^4$ | $R^5$ | $R^6$ | $R^7$ | Q |
|---|---|---|---|---|---|---|---|
| H | $OCH_2CH=NOCH_3$ | Cl | H | F | $-(CH_2)_3-$ | | O |
| H | $OCH_2CH=NOCH_2CH=CH_2$ | Cl | H | F | $-(CH_2)_3-$ | | O |
| H | $OCH_2CH=NOCH_2COOCH_3$ | Cl | H | F | $-(CH_2)_3-$ | | O |
| H | $OCH_2COOCH_2COOCH_3$ | Cl | H | F | $-(CH_2)_3-$ | | O |
| H | $OCF_2CHFCl$ | Cl | H | F | $-(CH_2)_3-$ | | O |
| H | $OCF_2CHF_2$ | Cl | H | F | $-(CH_2)_4-$ | | O |
| H | $OCH_2CH=CHCl$ | Cl | H | F | $-(CH_2)_3-$ | | O |
| H | $OCF_3$ | Cl | H | F | $-(CH_2)_3-$ | | O |
| H | $OCF_2Cl$ | Cl | H | F | $-(CH_2)_3-$ | | O |
| H | $-N-CO-CH_2-O-$<br>\|<br>$CH_2CH=CH_2$ | | H | H | $-(CH_2)_3-$ | | O |
| H | $-N-CO-CH_2-O-$<br>\|<br>$CH_2CH=CH_2$ | | H | F | $-(CH_2)_3-$ | | O |
| H | $-N-CO-CH_2-O-$<br>\|<br>$CH_2C≡CH$ | | H | H | $-(CH_2)_3-$ | | O |

<u>Tabelle 1</u> - Fortsetzung

| $R^1$ | $R^2$ | $R^3$ | $R^4$ | $R^5$ | $R^6$ | $R^7$ | Q |
|---|---|---|---|---|---|---|---|
| H | $-N-CO-CH_2-O-$<br>\|<br>$CH_2C\equiv CH$ | | H | F | $-(CH_2)_3-$ | | O |
| H | $OCH_3$ | $CH_3$ | H | F | $-(CH_2)_3-$ | | O |
| H | $OCH_2CH=CH_2$ | $CH_3$ | H | F | $-(CH_2)_3-$ | | O |
| H | $OCH_2C\equiv CH$ | $CH_3$ | H | F | $-(CH_2)_3-$ | | O |
| H | $OCH_2COOCH_3$ | $CH_3$ | H | F | $-(CH_2)_3-$ | | O |
| H | $OCH_3$ | CN | H | F | $-(CH_2)_3-$ | | O |
| H | $OCH_2CH=CH_2$ | CN | H | F | $-(CH_2)_3-$ | | O |
| H | $OCH_2C\equiv CH$ | CN | H | F | $-(CH_2)_3-$ | | O |
| H | $OCH_2COOCH_3$ | CN | H | F | $-(CH_2)_3-$ | | O |
| H | $OCH_2CH_2OCH_3$ | CN | H | F | $-(CH_2)_3-$ | | O |
| H | $OCHF_2$ | CN | H | F | $-(CH_2)_4-$ | | O |
| H | $OCF_2CHFCl$ | CN | H | F | $-(CH_2)_3-$ | | O |
| H | $OCH_2CH=NOCH_3$ | CN | H | F | $-(CH_2)_3-$ | | O |
| H | $OC_4H_9$ | CN | H | F | $-(CH_2)_3-$ | | O |

Tabelle 1 - Fortsetzung

| $R^1$ | $R^2$ | $R^3$ | $R^4$ | $R^5$ | $R^6$ | $R^7$ | Q |
|---|---|---|---|---|---|---|---|
| H | $CF_3$ | Cl | H | F | $-(CH_2)_3-$ | | O |
| H | $CHF_2$ | Cl | H | F | $-(CH_2)_3-$ | | O |
| H | $CF_2Cl$ | Cl | H | F | $-(CH_2)_3-$ | | O |
| H | $OCH_3$ | Cl | H | F | $CH_3$ | Cl | O |
| H | $OCH_3$ | Cl | H | F | Cl | $CH_3$ | O |
| H | $OCH_3$ | Cl | H | F | Cl | H | O |
| H | $OCH_3$ | Cl | H | F | $CH_3$ | Br | O |
| H | $OCH_3$ | Cl | H | F | Br | $CH_3$ | O |
| H | $OCH_3$ | Cl | H | F | $-CH_2-C(CH_3)_2-CH_2-$ | | O |
| H | $OCH_3$ | Cl | H | F | $CH_3$ | $CH_3$ | O |
| H | $-NH-CO-CH_2-O-$ | | H | H | $CH_3$ | Cl | O |
| H | $-NH-CO-CH_2-O-$ | | H | H | Cl | $CH_3$ | O |
| H | $-NH-CO-CH_2-O-$ | | H | H | $-CH_2-C(CH_3)_2-CH_2-$ | | O |
| H | $-NH-CO-CH_2-O-$ | | H | H | $-CH_2-CH(CH_3)-CH_2-$ | | |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | Cl | Cl | H | Cl | $CH_3$ | $CH_3$ | O |
| H | Cl | Cl | H | F | $CH_3$ | $CH_3$ | O |
| H | Cl | $CF_3$ | H | Cl | $-(CH_2)_3-$ | | O |
| H | Cl | Br | H | Cl | $-(CH_2)_3-$ | | O |
| H | F | F | H | Cl | $-(CH_2)_3-$ | | O |
| H | $-N(CH_2CH=CH_2)-CO-CH_2-O-$ | | H | H | $-CH_2-C(CH_3)_2-CH_2-$ | | O |
| H | $-N(CH_2C\equiv CH)-CO-CH_2-O-$ | | H | F | $-CH_2-C(CH_3)_2-CH_2-$ | | O |
| H | $-N(CH_2CH=CH_2)-CO-CH_2-O-$ | | H | F | $-CH_2-C(CH_3)_2-CH_2-$ | | O |
| H | $-N(CH_2C\equiv CH)-CO-CH_2-O-$ | | H | H | $-CH_2-C(CH_3)_2-CH_2-$ | | O |
| H | $-N-CO-CH_2-O-$ 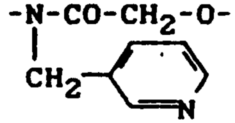 | | H | H | $CH_3$ | H | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | 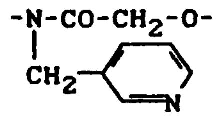 | | H | H | $CH_3$ | $CH_3$ | O |
| H | 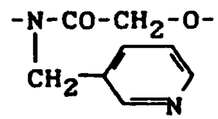 | | H | F | $CH_3$ | H | O |
| H | 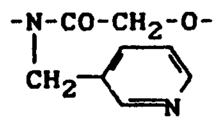 | | H | F | $CH_3$ | $CH_3$ | O |
| H | 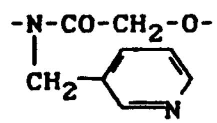 | | H | H | $-(CH_2)_3-$ | | O |
| H | 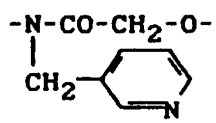 | | H | H | $-CH_2-C(CH_3)_2-CH_2-$ | | O |
| H | 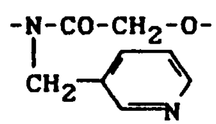 | | H | F | $-(CH_2)_3-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $-N-CO-CH_2-O-$<br>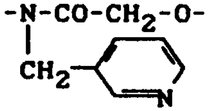 | | H | F | $-CH_2-C(CH_3)_2-CH_2-$ | | O |
| H | $-N-CO-CH_2-O-$<br>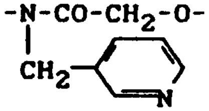 | | H | H | $-(CH_2)_4-$ | | O |
| H | $-N-CO-CH_2-O-$<br>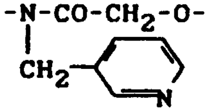 | | H | F | $-(CH_2)_4-$ | | O |
| H | $-N-CO-CH_2-O-$<br>$CH_2CH=CH_2$ | | H | H | $-(CH_2)_4-$ | | O |
| H | $-N-CO-CH_2-O-$<br>$CH_2CH=CH_2$ | | H | H | $CH_3$ | $CH_3$ | O |
| H | $-N-CO-CH_2-O-$<br>$CH_2CH=CH_2$ | | H | F | $-(CH_2)_4-$ | | O |
| H | $-N-CO-CH_2-O-$<br>$CH_2CH=CH_2$ | | H | H | $CH_3$ | $CH_3$ | O |

Tabelle 1 - Fortsetzung

| $R^1$ | $R^2$ | $R^3$ | $R^4$ | $R^5$ | $R^6$ | $R^7$ | Q |
|---|---|---|---|---|---|---|---|
| H | $-N-CO-CH_2-O-$<br>\|<br>$CH_2C\equiv CH$ | | H | H | $-(CH_2)_4-$ | | O |
| H | $-N-CO-CH_2-O-$<br>\|<br>$CH_2C\equiv CH$ | | H | H | $CH_3$ | $CH_3$ | O |
| H | $-N-CO-CH_2-O-$<br>\|<br>$CH_2C\equiv CH$ | | H | F | $-(CH_2)_4-$ | | O |
| H | $-N-CO-CH_2-O-$<br>\|<br>$CH_2C\equiv CH$ | | H | F | $CH_3$ | $CH_3$ | O |
| H | $-N-CO-CH_2-O-$<br>\|<br>$CH_2CH=CH_2$ | | H | H | Br | $CH_3$ | O |
| H | $-N-CO-CH_2-O-$<br>\|<br>$CH_2CH-CH_2$ | | H | F | Br | $CH_3$ | O |
| H | $-N-CO-CH_2-O-$<br>\|<br>$CH_2C\equiv CH$ | | H | H | Br | $CH_3$ | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $-N(CH_2C\equiv CH)-CO-CH_2-O-$ | | H | F | Br | $CH_3$ | O |
| H | 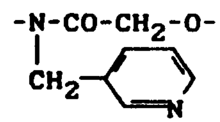 | | H | H | Br | $CH_3$ | O |
| H | 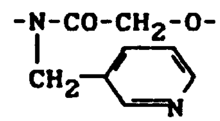 | | H | F | Br | $CH_3$ | O |
| H | $OCH(CH_3)-COOC_2H_5$ | $CH_3$ | H | F | $CH_3$ | H | O |
| H | $OCH(CH_3)-COOC_2H_5$ | $CH_3$ | H | F | $CH_3$ | $CH_3$ | O |
| H | $OCH(CH_3)-COOC_2H_5$ | $CH_3$ | H | F | $-(CH_2)_3-$ | | O |
| H | $OCH(CH_3)-COOC_2H_5$ | $CH_3$ | H | F | $-(CH_2)_4-$ | | O |

Tabelle 1 - Fortsetzung

| $R^1$ | $R^2$ | $R^3$ | $R^4$ | $R^5$ | $R^6$ | $R^7$ | Q |
|---|---|---|---|---|---|---|---|
| H | $OCH(CH_3)_2$ | $CH_3$ | H | F | $CH_3$ | $CH_3$ | O |
| H | $OCH(CH_3)_2$ | $CH_3$ | H | F | $-(CH_2)_3-$ | | O |
| H | $OCH(CH_3)_2$ | $CH_3$ | H | F | $-(CH_2)_4-$ | | O |
| H | $OCH_2C{\equiv}CH$ | $CH_3$ | H | F | $CH_3$ | $CH_3$ | O |
| H | $OCH_2C{\equiv}CH$ | $CH_3$ | H | F | $-(CH_2)_3-$ | | O |
| H | $OCH_2C{\equiv}CH$ | $CH_3$ | H | F | $-(CH_2)_4-$ | | O |
| H | $OCH_2CH{=}CH_2$ | $CH_3$ | H | F | $CH_3$ | $CH_3$ | O |
| H | $OCH_2CH{=}CH_2$ | $CH_3$ | H | F | $-(CH_2)_3-$ | | O |
| H | $OCH_2CH{=}CH_2$ | $CH_3$ | H | F | $-(CH_2)_4-$ | | O |
| H | $OCH_2COO$ 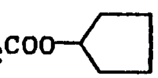 | $CH_3$ | H | F | $CH_3$ | $CH_3$ | O |
| H | $OCH_2COO$ 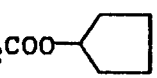 | $CH_3$ | H | F | $-(CH_2)_3-$ | | O |
| H | $OCH_2COO$ 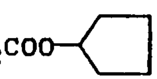 | $CH_3$ | H | F | $-(CH_2)_4-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | $SCH_2COO$ 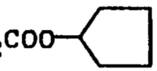 | $CH_3$ | H | F | $CH_3$ | $CH_3$ | O |
| H | $SCH_2COO$ 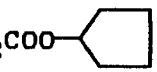 | $CH_3$ | H | F | $-(CH_2)_3-$ | | O |
| H | $SCH_2COO$ 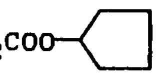 | $CH_3$ | H | F | $-(CH_2)_4-$ | | O |
| H | $OCH_2C≡CH$ | CN | H | H | $CH_3$ | $CH_3$ | O |
| H | $OCH_2C≡CH$ | CN | H | F | $CH_3$ | $CH_3$ | O |
| H | $OCH_2C≡CH$ | CN | H | H | $-(CH_2)_4-$ | | O |
| H | $OCH_2C≡CH$ | CN | H | F | $-(CH_2)_4-$ | | O |
| H | $OCH_2CH=CH_2$ | CN | H | H | $CH_3$ | $CH_3$ | O |
| H | $OCH_2CH=CH_2$ | CN | H | F | $CH_3$ | $CH_3$ | O |
| H | $OCH_2CH=CH_2$ | CN | H | H | $-(CH_2)_4-$ | | O |
| H | $OCH_2CH=CH_2$ | CN | H | F | $-(CH_2)_4-$ | | O |
| H | $(OCH_2CH_2)_2OC_2H_5$ | CN | H | H | $CH_3$ | H | O |

Tabelle 1 - Fortsetzung

| $R^1$ | $R^2$ | $R^3$ | $R^4$ | $R^5$ | $R^6$ | $R^7$ | Q |
|---|---|---|---|---|---|---|---|
| H | $(OCH_2CH_2)_2OC_2H_5$ | CN | H | F | $CH_3$ | H | O |
| H | $(OCH_2CH_2)_2OC_2H_5$ | CN | H | H | $CH_3$ | $CH_3$ | O |
| H | $(OCH_2CH_2)_2OC_2H_5$ | CN | H | F | $CH_3$ | $CH_3$ | O |
| H | $(OCH_2CH_2)_2OC_2H_5$ | CN | H | H | $-(CH_2)_3-$ | | O |
| H | $(OCH_2CH_2)_2OC_2H_5$ | CN | H | F | $-(CH_2)_3-$ | | O |
| H | $(OCH_2CH_2)_2OC_2H_5$ | CN | H | H | $-(CH_2)_4-$ | | O |
| H | $(OCH_2CH_2)_2OC_2H_5$ | CN | H | F | $-(CH_2)_4-$ | | O |
| H | $SCH(CH_3)_2$ | CN | H | F | $CH_3$ | H | O |
| H | $SCH(CH_3)_2$ | CN | H | F | $CH_3$ | $CH_3$ | O |
| H | $SCH(CH_3)_2$ | CN | H | F | $-(CH_2)_3-$ | | O |
| H | $SCH(CH_3)_2$ | CN | H | F | $-(CH_2)_4-$ | | O |
| H | $SCH_2CH=CH_2$ | CN | H | F | $CH_3$ | H | O |
| H | $SCH_2CH=CH_2$ | CN | H | F | $CH_3$ | $CH_3$ | O |
| H | $SCH_2CH=CH_2$ | CN | H | F | $-(CH_2)_3-$ | | O |
| H | $SCH_2CH=CH_2$ | CN | H | F | $-(CH_2)_4-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | -N(H)-CO-O- | | H | H | $CH_3$ | H | O |
| H | -N(H)-CO-O- | | H | H | $CH_3$ | $CH_3$ | O |
| H | -N(H)-CO-O- | | H | H | $-(CH_2)_3-$ | | O |
| H | -N(H)-CO-O- | | H | H | $-(CH_2)_4-$ | | O |
| H | -N(H)-CO-O- | | H | F | $CH_3$ | H | O |
| H | -N(H)-CO-O- | | H | F | $CH_3$ | $CH_3$ | O |
| H | -N(H)-CO-O- | | H | F | $-(CH_2)_3-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | -N-CO-O-<br>H | | H | F | $-(CH_2)_4-$ | | O |
| H | -N-CO-O-<br>$CH_2C\equiv CH$ | | H | H | $CH_3$ | H | O |
| H | -N-CO-O-<br>$CH_2C\equiv CH$ | | H | H | $CH_3$ | $CH_3$ | O |
| H | -N-CO-O-<br>$CH_2C\equiv CH$ | | H | H | $-(CH_2)_3-$ | | O |
| H | -N-CO-O-<br>$CH_2C\equiv CH$ | | H | H | $-(CH_2)_4-$ | | O |
| H | -N-CO-O-<br>$CH_2C\equiv CH$ | | H | F | $CH_3$ | H | O |
| H | -N-CO-O-<br>$CH_2C\equiv CH$ | | H | F | $CH_3$ | $CH_3$ | O |

**Tabelle 1 - Fortsetzung**

| $R^1$ | $R^2$ | $R^3$ | $R^4$ | $R^5$ | $R^6$ | $R^7$ | Q |
|---|---|---|---|---|---|---|---|
| H | $-N(CH_2C{\equiv}CH)-CO-O-$ | | H | F | $-(CH_2)_3-$ | | O |
| H | $-N(CH_2C{\equiv}CH)-CO-O-$ | | H | F | $-(CH_2)_4-$ | | O |
| H | $-N(CH_2CH{=}CH_2)-CO-O-$ | | H | H | $CH_3$ | H | O |
| H | $-N(CH_2CH{=}CH_2)-CO-O-$ | | H | H | $CH_3$ | $CH_3$ | O |
| H | $-N(CH_2CH{=}CH_2)-CO-O-$ | | H | H | $-(CH_2)_3-$ | | O |
| H | $-N(CH_2CH{=}CH_2)-CO-O-$ | | H | H | $-(CH_2)_4-$ | | O |
| H | $-N(CH_2CH{=}CH_2)-CO-O-$ | | H | F | $CH_3$ | H | O |

**Tabelle 1 - Fortsetzung**

| $R^1$ | $R^2$ | $R^3$ | $R^4$ | $R^5$ | $R^6$ | $R^7$ | Q |
|---|---|---|---|---|---|---|---|
| H | $-N(CH_2CH=CH_2)-CO-O-$ | | H | F | $CH_3$ | $CH_3$ | O |
| H | $-N(CH_2CH=CH_2)-CO-O-$ | | H | F | $-(CH_2)_3-$ | | O |
| H | $-N(CH_2CH=CH_2)-CO-O-$ | | H | F | $-(CH_2)_4-$ | | O |
| H | $-N(H)-CO-S-$ | | H | H | $CH_3$ | H | O |
| H | $-N(H)-CO-S-$ | | H | H | $CH_3$ | $CH_3$ | O |
| H | $-N(H)-CO-S-$ | | H | H | $-(CH_2)_3-$ | | O |
| H | $-N(H)-CO-S-$ | | H | H | $-(CH_2)_4-$ | | O |

Tabelle 1 - Fortsetzung

| $R^1$ | $R^2$ | $R^3$ | $R^4$ | $R^5$ | $R^6$ | $R^7$ | Q |
|---|---|---|---|---|---|---|---|
| H | -N-CO-S-<br>H | | H | F | $CH_3$ | H | O |
| H | -N-CO-S-<br>H | | H | F | $CH_3$ | $CH_3$ | O |
| H | -N-CO-S-<br>H | | H | F | $-(CH_2)_3-$ | | O |
| H | -N-CO-S-<br>H | | H | F | $-(CH_2)_4-$ | | O |
| H | -N-CO-S-<br>$CH_2C\equiv CH$ | | H | H | $CH_3$ | H | O |
| H | -N-CO-S-<br>$CH_2C\equiv CH$ | | H | H | $CH_3$ | $CH_3$ | O |
| H | -N-CO-S-<br>$CH_2C\equiv CH$ | | H | H | $-(CH_2)_3-$ | | O |

**Tabelle 1** - Fortsetzung

| $R^1$ | $R^2$ | $R^3$ | $R^4$ | $R^5$ | $R^6$ | $R^7$ | Q |
|---|---|---|---|---|---|---|---|
| H | -N-CO-S-<br>\|<br>$CH_2C{\equiv}CH$ | | H | H | $-(CH_2)_4-$ | | O |
| H | -N-CO-S-<br>\|<br>$CH_2C{\equiv}CH$ | | H | F | $CH_3$ | H | O |
| H | -N-CO-S-<br>\|<br>$CH_2C{\equiv}CH$ | | H | F | $CH_3$ | $CH_3$ | O |
| H | -N-CO-S-<br>\|<br>$CH_2C{\equiv}CH$ | | H | F | $-(CH_2)_3-$ | | O |
| H | -N-CO-S-<br>\|<br>$CH_2C{\equiv}CH$ | | H | F | $-(CH_2)_4-$ | | O |
| H | -N-CO-S-<br>\|<br>$CH_2CH{=}CH_2$ | | H | H | $CH_3$ | H | O |
| H | -N-CO-S-<br>\|<br>$CH_2CH{=}CH_2$ | | H | H | $CH_3$ | $CH_3$ | O |

**Tabelle 1 - Fortsetzung**

| $R^1$ | $R^2$ | $R^3$ | $R^4$ | $R^5$ | $R^6$ | $R^7$ | Q |
|---|---|---|---|---|---|---|---|
| H | $-N(CH_2CH{=}CH_2)-CO-S-$ | | H | H | $-(CH_2)_3-$ | | O |
| H | $-N(CH_2CH{=}CH_2)-CO-S-$ | | H | H | $-(CH_2)_4-$ | | O |
| H | $-N(CH_2CH{=}CH_2)-CO-S-$ | | H | F | $CH_3$ | H | O |
| H | $-N(CH_2CH{=}CH_2)-CO-S-$ | | H | F | $CH_3$ | $CH_3$ | O |
| H | $-N(CH_2CH{=}CH_2)-CO-S-$ | | H | F | $-(CH_2)_3-$ | | O |
| H | $-N(CH_2CH{=}CH_2)-CO-S-$ | | H | F | $-(CH_2)_4-$ | | O |

Verwendet man beim erfindungsgemäßen Verfahren (a) beispielsweise 2-Fluor-4-methyl-5-propargyloxy-phenylisocyanat und 2,2,6-Trimethyl-1,3-dioxin-4-on als Ausgangsstoffe, so kann der Reaktionsablauf durch das folgende Formelschema wiedergegeben werden:

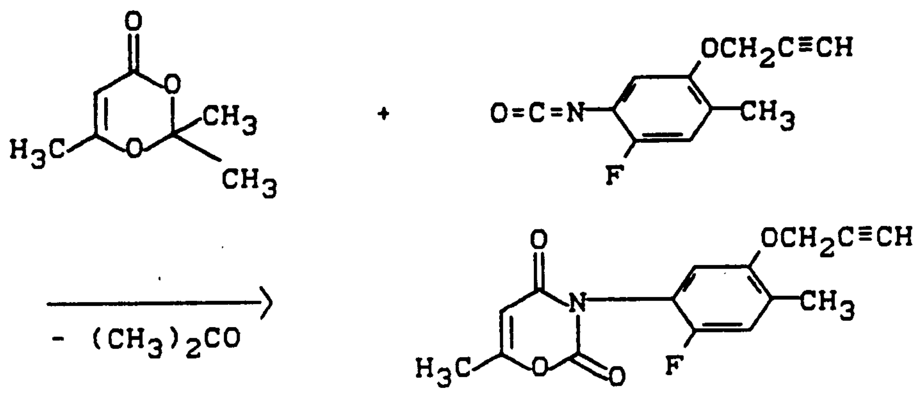


Verwendet man beim erfindungsgemäßen Verfahren (b) beispielsweise Methylmalonsäure-dichlorid und 6-Isocyanato-1,4-benzodioxan als Ausgangsstoffe, so kann der Reaktionsverlauf durch das folgende Formelschema wiedergegeben werden:

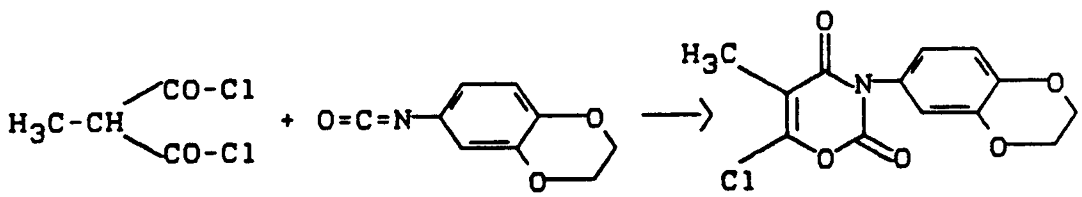

Verwendet man beim erfindungsgemäßen Verfahren (c) beispielsweise 2-Fluor-4-chlor-5-isopropoxy-phenylisocyanat und 2-Diazo-cyclohexan-1,3-dion als Ausgangsstoffe, so kann der Reaktionsablauf durch das folgende Formelschema wiedergegeben werden:

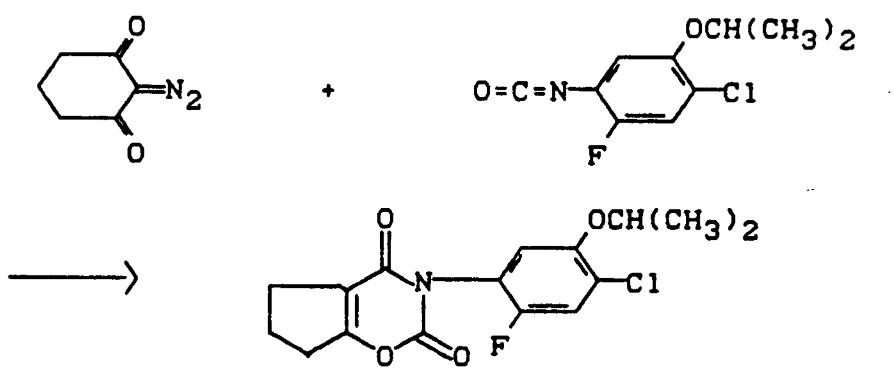

Verwendet man beim erfindungsgemäßen Verfahren (d) beispielsweise 2-Fluor-4-chlor-5-propargyloxy-phenylisocyanat und Salicylsäure-methylester als Ausgangsstoffe, so kann der Reaktionsablauf durch das folgende Formelschema wiedergegeben werden:

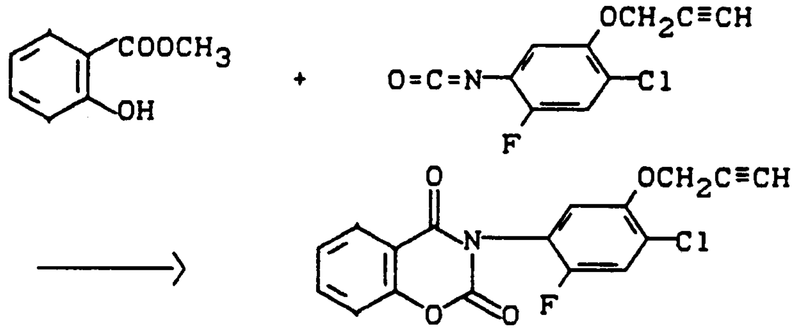

Die bei den erfindungsgemäßen Verfahren (a), (b), (c) und (d) zur Herstellung von Verbindungen der Formel (I) als Ausgangsstoffe benötigten Aryliso(thio)cyanate sind durch die Formel (II) allgemein definiert.

In Formel (II) haben $R^1$, $R^2$, $R^3$, $R^4$, $R^5$ und Q vorzugsweise bzw. insbesondere die gleichen Bedeutungen, wie sie bereits im Zusammenhang mit der Beschreibung der erfindungsgemäßen Verbindungen der Formel (I) vorzugsweise bzw. als insbesondere bevorzugt angegeben sind.

Als Beispiele für die Ausgangsstoffe der Formel (II) seien genannt:
2-Fluor-, 3-Fluor-, 4-Fluor- und 2,4-Difluor-phenyliso cyanat bzw. -phenylisothiocyanat, 2-Chlor-, 3-Chlor-, 4-chlor-, 2,3-Dichlor-, 2,4-Dichlor- und 3,4-Dichlorphenylisocyanat bzw. -phenylisothiocyanat, 3-Brom- und 4-Brom-phenylisocyanat bzw. -phenylisothiocyanat, 3-Trifluormethyl- und 4-Trifluormethyl-phenylisocyanat bzw. -phenylisothiocyanat, 3-Chlor-4-trifluormethyl- und 4-Chlor-3-trifluormethyl-phenylisocyanat bzw. -phenylisothiocyanat, 3-Chlor-4-methyl- und 4-Chlor-3-methyl-phenylisocyanat bzw. -phenylisothiocyanat, 3-Methoxy-, 3-Ethoxy-, 4-Methoxy-, 4-Ethoxy- und 3-Isopropoxy-phenylisocyanat bzw. -phenylisothiocyanat, 4-Nitro-phenylisocyanat und 4-Nitro-phenylisothiocyanat, 2,4-Dichlor-5-methoxy-, 2,4-Dichlor-5-ethoxy- und 2,4-Dichlor-5-isopropoxy-phenylisocyanat bzw, -phenylisothiocyanat, 2-Fluor-4-chlor-5-methoxycarbonylmethoxy-phenylisocyanat bzw. -phenylisothiocyanat, 2,4-Dichlor-5-methoxycarbonylmethoxy-phenylisocyanat bzw. -phenylisothiocyanat, 3-Methoxy-4-chlor-phenylisocyanat bzw. -phenylisothiocyanat, 3-Isopropoxy-4-chlor-phenylisocyanat bzw. -phenylisothiocyanat, 3-(1-Methoxycarbonyl)-ethoxy-4-chlorphenylisocyanat bzw. -phenylisothiocyanat, 2,4-Dichlor-5-(1-methoxycarbonyl)-ethoxy-phenylisocyanat bzw. -phenylisothiocyanat, 3-Allyloxy-4-chlor-phenylisocyanat bzw. -phenylisothiocyanat, 2-Fluor-4-chlor-5-allyloxyphenyl-isocyanat bzw. -phenylisothiocyanat, 2,4-Dichlor-5-allyloxy-phenylisocyanat bzw. -phenylisothiocyanat, 3-Propargyloxy-4-chlor-phenylisocyanat bzw. -phenylisothiocyanat, 2,4-Dichlor-5-propargyloxy-phenylisocyanat bzw. -phenylisothiocyanat, 2-Fluor-4-chlor-5-propargyloxy-phenylisocyanatbzw. -phenylisothiocyanat.

Die Ausgangsstoffe der Formel (II) sind teilweise bekannt und können nach an sich bekannten Verfahren hergestellt werden (vgl. EP-A 70 389, EP-A 69 855, EP-A 75 267, EP-A 230 874, EP-A 255 601, EP-A 263 299).

Man erhält die Aryliso(thio)cyanate der Formel (II), wenn man Arylamine der allgemeinen Formel (X)

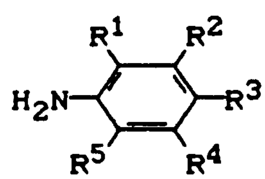 (X)

in welcher

$R^1$, $R^2$, $R^3$, $R^4$ und $R^5$ die oben angegebenen Bedeutungen haben,

mit Phosgen bzw. mit Thiophosgen, gegebenenfalls in Gegenwart eines Verdünnungsmittels, wie z. B. Toluol bzw. Chloroform und Wasser, bei Temperaturen zwischen -10 °C und 120 °C umsetzt.

Die Arylamine der allgemeinen Formel (X) sind bekannt und/oder können nach an sich bekannten Verfahren hergestellt werden (vgl. EP-A 61 741, EP-A 69 855, EP-A 70 389, EP-A 75 267, EP-A 83 055, EP-A 95 192, EP-A 170 191, EP-A 211 805, EP-A 218 972, EP-A 237 899, DE-OS 27 40 836).

Die beim erfindungsgemäßen Verfahren (a) zur Herstellung von Verbindungen der Formel (I) weiter als Ausgangsstoffe zu verwendenden 1,3-Dioxin-4-one sind durch die Formel (III) allgemein definiert.

In Formel (III) haben $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^6$ und $R^7$ angegeben wurden, und $R^9$ und $R^{10}$ stehen vorzugsweise für Wasserstoff oder $C_1$-$C_6$-Alkyl, insbesondere für Methyl.

Beispiele für die Ausgangsstoffe der Formel (III) sind in der nachstehenden Tabelle 2 aufgeführt:

Tabelle 2: Beispiele für die Ausgangsstoffe der Formel (III)

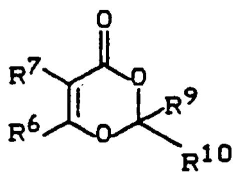

(III)

| $R^6$ | $R^7$ | $R^9$ | $R^{10}$ |
|---|---|---|---|
| $CH_3$ | H | $CH_3$ | $CH_3$ |
| $-(CH_2)_3-$ | | $CH_3$ | $CH_3$ |
| $-(CH_2)_4-$ | | $CH_3$ | $CH_3$ |
| $-CH_2-C(CH_3)_2-CH_2-$ | | $CH_3$ | $CH_3$ |
| $CH_3$ | H | H | $C_2H_5$ |
| $CH_3$ | H | H | $C_3H_7$ |
| $CH_3$ | H | $CH_3$ | $C_2H_5$ |
| $-(CH_2)_3-$ | | $CH_3$ | $C_2H_5$ |
| $CH_3$ | $CH_3$ | $CH_3$ | $CH_3$ |
| 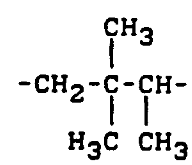 | | $CH_3$ | $CH_3$ |
| $CH_3$ | $C_2H_5$ | $CH_3$ | $CH_3$ |

Die Ausgangsstoffe der Formel (III) sind bekannt und/oder können nach an sich bekannten Verfahren hergestellt werden (vgl. Chem. Ber. 105 (1972), 137 - 149; J. Am. Chem. Soc. 74 (1952), 6305 - 6306; loc. cit. 75 (1953), 5400 - 5402; DE-OS 19 57 312).

Die beim erfindungsgemäßen Verfahren (b) zur Herstellung von Verbindungen der Formel (I) als Ausgangsstoffe zu verwendenden Dicarbonsäure-dichloride sind durch die Formel (IV) allgemein definiert.

In Formel (IV) hat $R^7$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^7$ angegeben wurden.

Als Beispiele für die Ausgangsstoffe der Formel (IV) seien genannt:
Malonsäure-dichlorid, Methylmalonsäure-dichlorid und Ethylmalonsäure-dichlorid.

Die Dicarbonsäure-dichloride der Formel (IV) sind bekannte organische Synthesechemikalien.

Die beim erfindungsgemäßen Verfahren (c) zur Herstellung von Verbindungen der Formel (I) als Ausgangsstoffe zu verwendenden 2-Diazo-1,3-diketone sind durch die Formel (V) allgemein definiert.

In Formel (V) haben $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^6$ und $R^7$ angegeben wurden.

Als Beispiele für die Ausgangsstoffe der Formel (V) seien genannt:
2-Diazo-butan-1,3-dion, 3-Diazo-pentan-2,4-dion, 3-Diazo-hexan-2,4-dion, 2-Diazo-cyclopentan-1,3-dion, 2-Diazo-cyclohexan-1,3-dion, 2-Diazo-cycloheptan-1,3-dion und 2-Diazo-5,5-dimethyl-cyclohexan-1,3-dion.

Die Ausgangsstoffe der Formel (V) sind bekannt und/oder können nach an sich bekannten Verfahren hergestellt werden (vgl. Liebigs Ann. Chem. 687 (1965), 214 - 231; Chem. Ber. 99 (1966), 3128 - 3147; loc. cit. 104 (1971), 1942 - 1956).

Die beim erfindungsgemäßen Verfahren (d) zur Herstellung von Verbindungen der Formel (I) als Ausgangsstoffe zu verwendende Salicylsäure und deren Ester sind durch die Formel (VI) allgemein definiert.

In Formel (VI) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde.

Als Beispiele für die Ausgangsstoffe der Formel (VI) seien genannt:
Salicylsäure sowie deren Methylester und Ethylester.

Die Ausgangsstoffe der Formel (VI) sind bekannte organische Synthesechemikalien.

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen vorzugsweise hochsiedende, inerte organische Lösungsmittel in Betracht. Hierzu gehören vor allem, gegebenenfalls halogenierte, Kohlenwasserstoffe, wie Dekalin, Tetralin, Toluol, Xylol, ferner Chlorbenzol und 1,2-Dichlorbenzol sowie Mesitylen.

Die Reaktionstemperaturen können bei dem erfindungsgemäßen Verfahren (a) in einem größeren Bereich variiert werden. Im allgemeinen arbeitet man bei Temperaturen zwischen 80 °C und 250 °C, vorzugsweise bei Temperaturen zwischen 120 °C und 220 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel - gegebenenfalls aber auch ohne Verdünnungsmittel - durchgeführt und das Reaktionsgemisch wird bei der jeweils erforderlichen Temperatur gerührt. Die Aufarbeitung erfolgt bei dem erfindungsgemäßen Verfahren (a) jeweils nach üblichen Methoden (vgl. auch die Herstellungsbeispiele).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden gegebenenfalls in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Das erfindungsgemäße Verfahren (c) zur Herstellung der neuen Verbindungen der Formel (I) wird gegebenenfalls unter Verwendung von Verdünnungsmitteln durchgeführt. Als Verdünnungsmittel kommen die gleichen inerten organischen Lösungsmittel vorzugsweise in Betracht, die oben für Verfahren (a) angegeben sind.

Die Reaktionstemperaturen können bei dem erfindungsgemäßen Verfahren (c) in einem größeren Bereich variiert werden. Im allgemeinen arbeitet man bei Temperaturen zwischen 80 °C und 250 °C, vorzugsweise bei Temperaturen zwischen 120 °C und 220 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden gegebenenfalls in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden (vgl. auch die Herstellungsbeispiele).

Das erfindungsgemäße Verfahren (d) zur Herstellung der neuen Verbindungen der Formel (I) wird gegebenenfalls in Gegenwart eines Verdünnungmittels durchgeführt. Als Verdünnungsmittel kommen vorzugsweise die gleichen inerten organischen Lösungsmittel in Betracht, die oben für Verfahren (a) angegeben sind.

Als Säureakzeptoren können bei dem erfindungsgemäßen Verfahren (d) alle üblicherweise für derartige Umsetzungen verwendbaren Säurebindemittel eingesetzt werden.

Vorzugsweise in Frage kommen aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (d) in einem größeren Bereich variiert werden. Im allgemeinen arbeitet man bei Temperaturen zwischen 0 °C und 250 °C, vorzugsweise bei Temperaturen zwischen 10 °C und 200 °C.

Das erfindungsgemäße Verfahren (d) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur

selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, vor allem im Nachauflauf-Verfahren.

In gewissem Umfang zeigen sie auch fungizide Wirkung, z. B. gegen Phytophtora-Arten.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)harnstoff (ISOPROTURON); (2-Methyl-4-

chlorphenoxy)essigsäure (MCPA); (4-Chlor-2-methylphenoxy)-propionsäure (MCPP); N-Methyl-2-(1,3-benzthiazol-2-yloxy)-acetanilid (MEFENACET); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

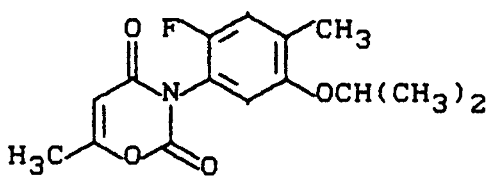

(Verfahren (a))

Ein Gemisch aus 1,42 g (0,01 Mol) 2,2,6-Trimethyl-4H-1,3-dioxin-4-on und 2,09 g (0,01 Mol) 2-Fluor-4-methyl-5-isopropoxy-phenylisocyanat wird 3 Stunden auf 140 °C erhitzt, wobei Aceton überdestilliert. Nach Abkühlen saugt man den Niederschlag ab.

Man erhält 0,7 g (23 % der Theorie) 3,4-Dihydro-3-(2-Fluor-4-methyl-5-isopropoxy-phenyl)-6-methyl-2H-1,3-oxazin-2,4-dion vom Schmelzpunkt 122 °C - 124 °C.
$^1$H-NMR (80 Mhz, $CDCl_3$, δ): 7.01 (d, 1H, J = 9,0 Hz); 6.66 (d, 1H, J = 5,0 Hz), 4.40 (sept. 1H, J = 5,9 Hz); 2.26 (s, 3H); 2.22 (s, 3H); 1.32 (d, 6H, J = 5.8 Hz).

Beispiel 2

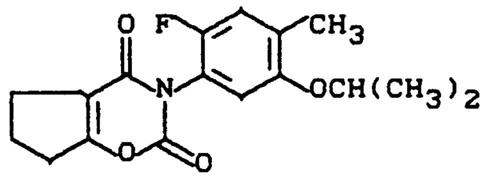

(Verfahren (a))

Eine Lösung von 1,68 g (0,01 Mol) 6,7-Dihydro-5H-cyclopenta-1,3-dioxin-4-on und 2,09 g (0,01 Mol) 2-Fluor-4-methyl-5-isopropoxyphenylisocyanat in 5 ml o-Xylol wird eine Stunde auf 150 °C erhitzt.

Nach Entfernung der flüchtigen Komponente isoliert man durch Säulenchromatographie über Kieselgel 0,91 g (28,5 % der Theorie) 3-(2-Fluor-4-methyl-5-isopropoxyphenyl)-2,3,4,5,6,7-hexahydrocyclopenta[e]-1,3-oxazin-2,4-dion vom Schmelzpunkt 131 °C - 133 °C.

$^1$H-NMR (80 Mhz, $CDCl_3$, δ): 7.01 (d, 1H, J = 9,0 Hz); 6.65 (d, 1H, J = 5,0 Hz); 4.39 (sept., 1H, J = 5,8 Hz); 2.85 (m, 2H); 2.76 (m, 2H); 2.22 (s, 3H), 2.16 (m, 2H); 1.32 (d, 6H, J = 5,6 Hz).

Beispiel 3

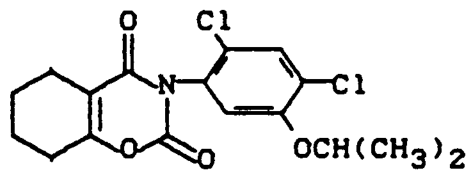

(Verfahren (a))

Eine Lösung von 5 g (0,027 Mol) 2,2-Dimethyl-4,5,6,7,8-hexahydro-cyclohexa-1,3-dioxin-4-on und 6,7 g (0,027 Mol) 2,4-Dichlor-5-isopropyloxy-phenylisocyanat in 20 ml Mesitylen wird 30 Minuten auf 210 °C erhitzt. Dabei destilliert man das Mesitylen ab.

Nach säulenchromatographischer Reinigung an Kieselgel mit dem Laufmittel Petrolether : Essigsäureethylester 5 : 1 erhält man 2,1 g (21 % der Theorie) 2,3,4,5,6,7,8-Heptahydro-3-(2,4-dichlor-5-isopropoxy-phenyl )-cyclohexa[e]-1,3-oxazin-2,4-dion als öligen Rückstand.

$^1$H-NMR (200 Mhz, $CDCl_3$, δ): 1.36 (d, 6H); 1.68 - 1.95 und 2.38 - 2.55 (m, 8H); 4.50 (m, 1H); 6,84 (s, 1H); 7.53 (s, 1H).

Beispiel 4

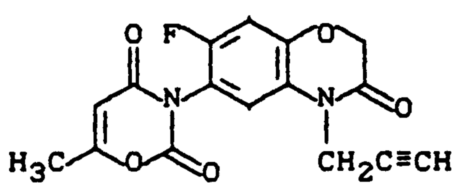

(Verfahren (a))

Man tropft 2,84 g (0,02 Mol) 2,2,6-Trimethyl-4H-1,3-dioxin-4-on zu einer auf 150 °C vorgeheizten Lösung von 4,92 g (0,02 Mol) 7-Fluor-6-isocyanato-4-propargyl-3,4-dihydro-3-oxo-2H-1,4-benzoxazin in 10

ml o-Dichlorbenzol. Man erhitzt die Reaktionsmischung 4 Stunden bei dieser Temperatur, kühlt ab und engt ein. Der Rückstand wird durch Säulenchromatographie über Kieselgel aufgetrennt.

Man erhält 1,68 g (25,5 % der Theorie) 3,4-Dihydro-3-(7-fluor-4-propargyl-3,4-dihydro-3-on-2H-1,4-benzoxazin-6-yl)-6-methyl-2H-1,3-oxazin-2,4-dion vom Schmelzpunkt 244 °C - 248 °C.
$^1$H-NMR (80 Mhz, $CDCl_3$/DMSO, δ): 7.36 (d, 1H, J = 6,6 Hz); 7.05 (d, 1H, J = 9,0 Hz); 6.09 (s, 1H); 4.77 (s, 2H); 4.65 (d, 2H, J = 2,0 Hz); 3.01 (t, 1H, J = 2,0 Hz); 2.28 (s, 3H).

Beispiel 5

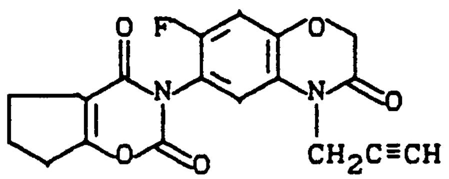

(Verfahren (a))

3,36 g (0,02 Mol) 6,7-Dihydro-5H-cyclopenta-1,3-dioxin-4-on und 4,92 g (0,02 Mol) 7-Fluor-6-isocyanato-4-propargyl-3,4-dihydro-3-oxo-2H-1,4-benzoxazin in 10 ml o-Dichlorbenzol werden 4 Stunden bei 150 °C erhitzt. Nach Abkühlen und Einengen wird der Rückstand säulenchromatographisch aufgetrennt.

Man isoliert 1,58 g (22,2 % der Theorie) 3-(7-Fluor-4-propargyl-3,4-dihydro-3-oxo-2H-1,4-benzoxazin-6-yl)-2,3,4,5,6,7-hexahydrocyclopenta[e]-1,3-oxazin-2,4-dion vom Schmelzpunkt 222 °C - 224 °C.
$^1$H-NMR (80 Mhz, $CDCl_3$, δ): 7.08 (d, 1H, J = 6,6 Hz); 6.90 (d, 1H, J = 9,0 Hz); 4.70 (dd, 1H, J = 17,3 Hz, J' = 2,0 Hz); 4,6 g (s, 2H); 4.61 (dd, 1H, J = 17,3 Hz, J' = 2,0 Hz); 2.87 (m, 2H); 2.77 (m, 2H); 2.29 (t, 1H, J = 2,0 Hz); 2.21 (m, 2H).

Beispiel 6

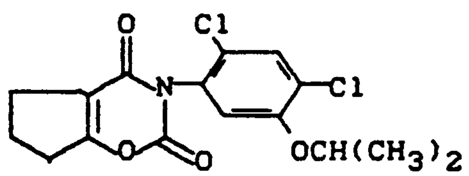

(Verfahren (c))

Eine Lösung aus 10 g (75 mMol) 2-Diazo-cyclohexa-1,3-dion und 18,3 g (75 mMol) 2,4-Dichlor-5-isopropoxy-phenylisocyanat in 50 ml Xylol wird bei 140 °C zu 5 ml Xylol getropft. Nach beendeter Zugabe rührt man noch 10 Minuten bei 140 °C nach. Das Lösungsmittel wird abdestilliert und der Rückstand säulenchromatographisch an Kieselgel mit Petrolether : Essigsäuremethylester 5 : 1 gereinigt.

Man erhält 16,8 g (63 % der Theorie) 2,3,4,5,6,7-Hexahydro-3-[dichlor-5-isopropoxy-phenyl]-cyclopenta-[e]-1,3-oxazin-2,4-dion.
$^1$H-NMR (200 Mhz, $CDCl_3$, δ): 1.38 (d, 6H); 2.10 - 2.25 und 2.65 - 2.90 (m, 6H); 4.50 (m, 1H); 6.83 (s, 1H); 7.52 (s, 1H).

Beispiel 7

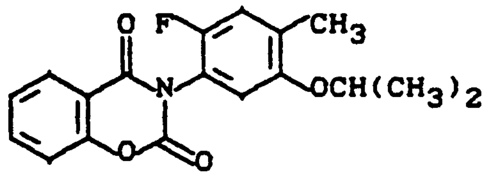

(Verfahren (d))

Man legt 2,76 g (0,02 Mol) Salicylsäure in 20 ml o-Dichlorbenzol vor, tropft 2,22 g (0,022 Mol) Triethylamin zu und rührt noch 30 Minuten bei Raumtemperatur nach. Anschließend gibt man 4,18 g (0,02 Mol) 2-Fluor-4-methyl-5-isopropoxy-phenylisocyanat zu und erhitzt 4 Stunden bei 150 °C.

Nach Einengen und säulenchromatographischer Reinigung erhält man 1,38 g (21 % der Theorie) 3-(2-Fluor-4-methyl-5-isopropoxyphenyl)-benzo[e]-1,3-oxazin-2,4-dion von Schmelzpunkt 118 °C - 122 °C.

$^1$H-NMR (80 Mhz, $CDCl_3$, $\delta$): 8.14 (dd, 1H, J = 8,0 Hz, J' = 1,0 Hz); 7.75 (dt, 1H, J = 8,0 Hz, J' = 1,0 Hz); 7.41 (dt, 1H, J = 8,0 Hz, J' = 1,0 Hz); 7.35 (d, 1H, J = 8,0 Hz); 7.05 (d, 1H, J = 9,0 Hz); 6.74 (d, 1H, J = 5,0 Hz); 4.42 (sept., 1H, J = 5,8 Hz); 2.25 (s, 3H); 1.33 (d, 6H, J = 5,8 Hz).

Analog zu den Beispielen 1 bis 7 und entsprechend der allgemeinen Beschreibung der erfindungsgemäßen Herstellungsverfahren können die in der nachstehenden Tabelle 3 aufgeführten Verbindungen der Formel (I) hergestellt werden.

**Tabelle 3: Herstellungsbeispiele für die Verbindungen der Formel (I)**

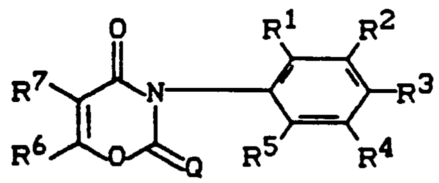

(I)

**Tabelle 3**

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 8 | H | $-O-CH_2CH_2-O-$ | | H | H | | $-(CH_2)_3-$ | O | Fp. 203 °C |
| 9 | H | $OCH_2C{\equiv}CH$ | Cl | H | F | | $-(CH_2)_3-$ | O | Fp. 131 °C |
| 10 | H | $-N(CH_2C{\equiv}CH)-CO-CH_2-O-$ | | H | F | | -CH=CH-CH=CH- | O | a) |
| 11 | H | $OCF_2Cl$ | Cl | H | F | | $-(CH_2)_3-$ | O | b) |
| 12 | H | $OCF_2CHFCl$ | Cl | H | F | | $-(CH_2)_4-$ | O | c) |
| 13 | H | $-O-CF_2-O-$ | | H | H | | $-(CH_2)_3-$ | O | Fp. 198 °C |

a) $^1$H-NMR (80 Mhz, $CDCl_3$, δ): 8.10 (dd, 1H, J = 8,0 Hz, J' = 1,0 Hz); 7.83 (dt, 1H, J = 8,0 Hz, J' = 1,0 Hz); 7.46 (t, 1H, J = 8,0 Hz); 7.41 (d, 1H, J = 8,0 Hz); 7.33 (d, 1H, J = 5,8 Hz); 6.98 (d, 1H, J = 9,0 Hz); 4.73 (s, 2H); 4.6 (dd, 2H).

b) $^1$H-NMR (80 Mhz, $CDCl_3$, δ): 7.41 (d, 1H, J = 9,5 Hz); 7.35 (d, 1H, J = 5,8 Hz); 2.95 - 2.70 (m, 4H); 2.27 - 2.10 (m, 2H).

c) $^1$H-NMR (80 Mhz, $CDCl_3$, δ): 7.39 (d, 1H, J = 9,5 Hz); 7.35 (d, 1H, J = 6,25 Hz); 6.45 und 6.20 (dt, 1H, J = 4,5 Hz, J' = 4,5 Hz); 2.55 - 2.37 und 1.65 - 1.45 (m, 8H).

<u>Tabelle 3</u> - Fortsetzung

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 14 | H | $-O-CF_2-CHF-O-$ | | H | F | | $-(CH_2)_3-$ | O | |
| 15 | H | $-O-CHF-CF_2-O-$ | | H | F | | $-(CH_2)_3-$ | O | |
| 16 | H | $-O-CH_2-O-CH_2-$ | | H | H | | $-(CH_2)_3-$ | O | Fp. 172 °C |
| 17 | H | $-N-CO-CH_2-O-$<br>\|<br>$CH_2C{\equiv}CH$ | | H | F | | $-(CH_2)_4-$ | O | $^1H$-NMR ($CDCl_3$): $\delta$ = 1,7(4); 2,3(4); 3,21(1); 4,62(2) |
| 18 | H | $OCF_2Cl$ | Cl | H | F | | $-(CH_2)_4-$ | O | |
| 19 | H | $OCH_2C{\equiv}CH$ | $CH_3$ | H | F | | $-(CH_2)_4-$ | O | |
| 20 | H | $-N-CO-CH_2-O-$<br>\|<br>$CH_2OCH_3$ | | H | H | | $-(CH_2)_4-$ | O | |
| 21 | H | $-N-CO-CH_2-O-$<br>\|<br>$CH_2C=CH_2$<br>\|<br>Cl | | H | H | $CH_3$ | $CH_3$ | O | |
| 22 | H | $OCH_2C{\equiv}CH$ | Cl | H | F | | $-(CH_2)_4-$ | O | |
| 23 | H | $-N-CO-CH_2-O-$<br>\|<br>$CH_2OCH_3$ | | H | F | | $-(CH_2)_4-$ | O | |

Tabelle 3 - Fortsetzung

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 24 | H | $OCH_2CH=CH_2$ | CN | H | F | | $-(CH_2)_4-$ | O | |
| 25 | H | $-N(CH_2OCH_3)-CO-CH_2-O-$ | | H | H | | $-(CH_2)_3-$ | O | Fp. 194-199° C |
| 26 | H | $OCH_2C{\equiv}CH$ | Cl | H | F | $CH_3$ | $CH_3$ | O | |
| 27 | H | $-N(CH_2OCH_3)-CO-CH_2-O-$ | | H | F | | $-(CH_2)_3-$ | O | Fp. 184 °C |
| 28 | H | $OCH_2CH=CH_2$ | CN | H | F | | $-(CH_2)_3-$ | O | |
| 29 | H | $-N(CH_2C{\equiv}CH)-CO-CH_2-O-$ | | H | H | | $-(CH_2)_4-$ | S | |
| 30 | H | $-N-CO-CH_2-O-$ 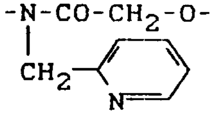 | | H | F | | $-(CH_2)_4-$ | O | Fp. 202-212° C |
| 31 | H | $-N(CH_2COOC_2H_5)-CO-CH_2-O-$ | | H | H | | $-(CH_2)_3-$ | O | Fp. 215-224° C |

Tabelle 3 - Fortsetzung

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 32 | H | $-N(CH_2COOC_2H_5)-CO-CH_2-O-$ | | H | H | $-(CH_2)_4-$ | | O | Fp. 214-221° C |
| 33 | H | $-N(CH_2CN)-CO-CH_2-O-$ | | H | H | $-(CH_2)_4-$ | | O | MS:[$M^+$] 353, 229, 161 |
| 34 | H | $-N(CH_2CN)-CO-CH_2-O-$ | | H | H | $-(CH_2)_3-$ | | O | Fp. 209° C |
| 35 | H | $-N(CH_2CH_2OC_2H_5)-CO-CH_2-O-$ | | H | H | $-(CH_2)_3$ | | O | Fp. 159-164° C |
| 36 | H | $-N(CH_2CH_2OC_2H_5)-CO-CH_2-O-$ | | H | H | $-(CH_2)_4-$ | | O | Fp. 151-155° C |
| 37 | H | $-N(CH_2OCH_3)-CO-CH_2-O-$ | | H | F | $CH_3$ | $CH_3$ | O | $^1H$-NMR ($CDCl_3$): $\delta$ = 1,95(3); 2,30(3); 3,38(3) 4,70(2) |

**Tabelle 3** - Fortsetzung

| Bsp.-Nr. | $R^1$ | $R^2$ | | $R^3$ | $R^4$ | $R^5$ | $R^6$ | $R^7$ | Q | Physikalische Daten |
|---|---|---|---|---|---|---|---|---|---|---|
| 38 | H | F | | CN | H | F | | $-(CH_2)_4-$ | O | Fp. 159-161° C |
| 39 | H | F | | CN | H | F | $CH_3$ | $CH_3$ | O | Fp. 235° C |
| 40 | H | | $-N-CO-CH_2-O-$<br>$CH_2CN$ | | H | F | | $-(CH_2)_4-$ | O | Fp. 220° C |
| 41 | H | | $-N-CO-CH_2-O-$<br>$CH_2CN$ | | H | F | | $-(CH_2)_3-$ | O | Fp. 209-211° C |
| 42 | H | | $-N-CO-CH_2-O-$<br>$CH_2COOC_2H_5$ | | H | F | | $-(CH_2)_4$ | O | $^1H$-NMR ($CDCl_3$):<br>$\delta$ = 1,25(3H);<br>1,80(4H);<br>2,45(4H) |
| 43 | H | | $-N-CO-CH_2-O-$<br>$CH_2COOC_2H_5$ | | H | F | | $-(CH_2)_3-$ | O | MS:[$M^+$] 404,<br>294, 221 |
| 44 | H | | $-N-CO-O-$ | | H | F | | $-(CH_2)_4-$ | O | MS:[$M^+$] 362,<br>238 |

<u>Tabelle 3</u> - Fortsetzung

| Bsp.-Nr. | $R^1$ | $R^2$ $R^3$ | $R^4$ | $R^5$ | $R^6$ $R^7$ | Q | Physikalische Daten |
|---|---|---|---|---|---|---|---|
| 45 | H | -N-CO-O-<br>\|<br>$CH_2C\equiv CH$ | H | F | $-(CH_2)_4-$ | O | MS:[$M^+$] 356, 330, 232 |
| 46 | H | -N-CO-O-<br>\|<br>$CH_2C\equiv CH$ | H | H | $-(CH_2)_4-$ | O | $^1$H-NMR ($CDCl_3$): δ = 1,80(4); 2,45(4); 4,60(2); 7,1-7,3(3) |
| 47 | H | $-N-CO-CH_2-O-$<br>\|<br>$CH_2CN$ | H | F | $CH_3$ $CH_3$ | O | Fp. 189° C |
| 48 | H | $-N-CO-CH_2-O-$<br>\|<br>$CH_2COOC_2H_5$ | H | F | $CH_3$ $CH_3$ | O | $^1$H-NMR ($CDCl_3$): δ = 1,25(3H); 1,95(3), 2,28(3); 4,20(2), 4,60(2) |
| 49 | H | -N-CO-O-<br>\|<br>$CH_2OCH_3$ | H | F | $CH_3$ $CH_3$ | O | MS:[$M^+$] 336, 238 |
| 50 | H | $-O-CF_2-O-$ | H | F | $-(CH_2)_4-$ | O | Fp. 125° C |

Tabelle 3 - Fortsetzung

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 51 | H | -N-CO-O-<br>\|<br>$CH_2CN$ | | H | F | | $-(CH_2)_4-$ | O | MS:[$M^+$] 357, 233, 125 |
| 52 | H | -N-CO-O-<br>\|<br>$CH_2CN$ | | H | F | | $-(CH_2)_3-$ | O | $^1H$-NMR ($CDCl_3$): δ = 4,75(2); 7,10(1); 7,25(1) |
| 53 | H | $OCH_2COOC_2H_5$ | Cl | H | F | | $-(CH_2)_4-$ | O | $^1H$-NMR ($CDCl_3$): δ = 1,25(3); 1,80(4); 2,45(4); 4,25(2) |
| 54 | H | $OCH_2COOC_2H_5$ | Cl | H | F | | $-(CH_2)_3-$ | O | $^1H$-NMR ($CDCl_3$): δ = 1,25(3); 2,20(2); 2,80(4); 4,25(2) |
| 55 | H | $OCH_2COOC_2H_5$ | Cl | H | F | $CH_3$ | $CH_3$ | O | Öl |
| 56 | H | -N-CO-S-<br>\|<br>$CH_2CN$ | | H | F | | $-(CH_2)_4-$ | O | MS:[$M^+$] 373 |

**Tabelle 3** - Fortsetzung

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 57 | H | $CH_3$<br>-N-CO-CH-O-<br>$CH_2C\equiv CH$ | | H | H | | $-(CH_2)_4-$ | O | Fp. 217-227° C |
| 58 | H | $-N-CO-CH_2-O-$<br>$CH_2CH=CH_2$ | | H | F | | $-(CH_2)_3-$ | O | Fp. 208° C |
| 59 | H | $-N-CO-CH_2-O-$<br>$CH_2CH=CH_2$ | | H | F | $CH_3$ | H | O | Fp. 196° C |
| 60 | H | $-N-CO-CH_2-O-$<br>$CH_2CH=CH_2$ | | H | F | $CH_3$ | $C_2H_5$ | O | Fp. 161° C |
| 61 | H | $-N-CO-CH_2-O-$<br>$CH_2C\equiv CH$ | | H | H | $CH_3$ | $C_2H_5$ | O | Fp. 278° C |
| 62 | H | $-N-CO-CH_2-O-$<br>$CH_2C\equiv CH$ | | H | H | $CH_3$ | H | O | Fp. 254° C |

**Tabelle 3 - Fortsetzung**

| Bsp.-Nr. | $R^1$ | $R^2$ | $R^3$ | $R^4$ | $R^5$ | $R^6$ | $R^7$ | Q | Physikalische Daten |
|---|---|---|---|---|---|---|---|---|---|
| 63 | H | $-N-CO-CH_2-O-$<br>$CH_2C\equiv CH$ | | H | F | $CH_3$ | $C_2H_5$ | O | Fp. 195° C |
| 64 | H | $O(CH_2CH_2O)_2C_2H_5$ | CN | H | F | $-(CH_2)_3-$ | | O | |
| 65 | H | $O(CH_2CH_2O)_2C_2H_5$ | CN | H | F | $CH_3$ | $C_2H_5$ | O | Fp. 77° C |
| 66 | H | $-N-CO-S-$<br>$CH_2C\equiv CH$ | | H | F | $-(CH_2)_3-$ | | O | Fp. 218° C |
| 67 | H | $-N-CO-S-$<br>$CH_2C\equiv CH$ | | H | F | $CH_3$ | $C_2H_5$ | O | Fp. 227° C |
| 68 | H | $-N-CO-S-$<br>$CH_2C\equiv CH$ | | H | F | $-(CH_2)_4-$ | | O | Fp. 139° C |
| 69 | H | $SCH_2COOC_2H_5$ | Cl | H | F | $-(CH_2)_3-$ | | O | |
| 70 | H | $SCH_2COOC_2H_5$ | Cl | H | F | $CH_3$ | $C_2H_5$ | O | |

## Anwendungsbeispiel

Im folgenden Anwendungsbeispiel wird die nachstehend aufgeführte Verbindung als Vergleichssubstanz herangezogen:

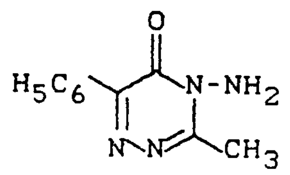

(A)

4-Amino-3-methyl-6-phenyl-4H-1,2,4-triazin-5-on (Metamitron, GOLTIX)
- bekannt aus DE-OS 23 64 474/Beispiel 1.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber der Vergleichsstubstanz (A) zeigen in diesem Test z.B. die Verbindungen gemäß den Herstellungsbeispielen: (2) und (3).

**Patentansprüche**

**1.** Herbizide Mittel, gekennzeichnet durch einen Gehalt an mindestens einem N-Phenyl-substituierten Oxazindion der Formel (IA)

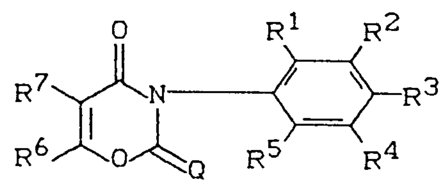

(IA)

in welcher

$R^1$ für Wasserstoff oder Halogen steht,

$R^2$ für Wasserstoff, Nitro, Cyano, Halogen steht oder für einen gegebenenfalls durch Fluor, Chlor, Brom, Cyano, $C_3$-$C_6$-Cycloalkyl, welches gegebenenfalls durch Fluor, Chlor, Brom, Methyl und/oder Ethyl substituiert ist, durch $C_1$-$C_6$-Alkoxy, $C_1$-$C_4$-Alkoxy-$C_1$-$C_4$-alkoxy, $C_1$-$C_6$-Alkylthio, $C_1$-$C_6$-Alkylsulfinyl, $C_1$-$C_6$-Alkylsulfonyl, $C_1$-$C_4$-Alkylthio-$C_1$-$C_4$-alkoxy, $C_1$-$C_4$-Alkylsulfonyl-$C_1$-$C_4$-alkoxy, $C_3$-$C_6$-Alkenyloxy, $C_3$-$C_6$-Alkinyloxy, $C_3$-$C_6$-Alkenylthio, $C_3$-$C_6$-Alkinylthio, Carboxy, $C_1$-$C_6$-Alkoxy-carbonyl, $C_3$-$C_6$-Alkenyloxy-carbonyl, $C_3$-$C_6$-Alkinyloxy-carbonyl, $C_3$-$C_6$-Cycloalkyloxy-carbonyl, $C_3$-$C_6$-Cycloalkenyloxy-carbonyl, $C_3$-$C_6$-Cycloalkyl-$C_1$-$C_4$-alkoxy-carbonyl, $C_3$-$C_6$-Cycloalkenyl-$C_1$-$C_4$-alkoxy-carbonyl, $C_1$-$C_4$-Alkoxy-$C_1$-$C_4$-alkoxy-carbonyl, $C_1$-$C_4$-Alkylthio-$C_1$-$C_4$-alkoxy-carbonyl, Benzyloxy-$C_1$-$C_4$-alkoxy-carbonyl, $C_1$-$C_4$-Alkoxy-carbonyl-$C_1$-$C_4$-alkoxy-carbonyl, $C_1$-$C_4$-Alkylamino-carbonyl-$C_1$-$C_4$-alkoxy-carbony, Di-($C_1$-$C_4$-alkyl)-amino-carbonyl-$C_1$-$C_4$-alkoxy-carbonyl, $C_1$-$C_4$-Alkoxy-$C_1$-$C_4$-alkyl-amino-carbonyl, $C_1$-$C_4$-Alkoxy-imino-$C_1$-$C_4$-alky, $C_3$-$C_4$-Alkenyloxy-imino-$C_1$-$C_4$-alkyl,

$C_1$-$C_4$-Alkoxy-carbonyl-$C_1$-$C_4$-alkoxy-imino-$C_1$-$C_4$-alkyl, $C_1$-$C_6$-Alkylthio-carbonyl, $C_3$-$C_6$-Alkenylthio-carbonyl, $C_3$-$C_6$-Alkinylthio-carbonyl, Carbamoyl, $C_1$-$C_6$-Alkylamino-carbonyl, $C_3$-$C_6$-Alkenylamino-carbonyl, $C_3$-$C_6$-Alkinylamino-carbonyl, Di-($C_1$-$C_4$-alkyl)-amino-carbonyl, Di-($C_3$-$C_4$-alkenyl)-amino-carbonyl und/oder Di-($C_3$-$C_4$-alkinyl)-amino-carbonyl substituierten Rest aus der Reihe $C_1$-$C_8$-Alkyl, $C_1$-$C_6$-Alkoxy, $C_1$-$C_6$-Alkylthio und $C_1$-$C_6$-Alkylamino oder für einen gegebenenfalls durch Fluor und/oder Chlor substituierten Rest aus der Reihe $C_3$-$C_6$-Alkenyl, $C_3$-$C_6$-Alkinyl, $C_3$-$C_6$-Alkenyloxy, $C_3$-$C_6$-Alkinyloxy, $C_3$-$C_6$-Alkenylthio, $C_3$-$C_6$-Alkinylthio, $C_1$-$C_6$-Alkylsulfinyl und $C_1$-$C_6$-Alkylsulfonyl steht,

$R^3$ für Wasserstoff, Halogen, Nitro, Cyano oder für einen gegebenenfalls durch Fluor und/oder Chlor substituierten Rest aus der Reihe $C_1$-$C_4$-Alkyl, $C_3$-$C_4$-Alkenyl, $C_3$-$C_4$-Alkinyl, $C_1$-$C_4$-Alkoxy, $C_3$-$C_4$-Alkenyloxy, $C_3$-$C_4$-Alkinyloxy, $C_1$-$C_4$-Alkylthio, $C_3$-$C_4$-Alkenylthio, $C_3$-$C_4$-Alkinylthio, $C_1$-$C_4$-Alkylsulfinyl, $C_1$-$C_4$-Alkylsulfonyl und $C_1$-$C_4$-Alkylamino steht, oder die beiden Reste

$R^2$ und $R^3$ zusammen für -O-$CH_2$-O-$CH_2$- oder die Gruppierung -X-$(CO)_n$-A-Y- stehen, worin

n für die Zahlen 0 oder 1 steht,

A für eine direkte Bindung oder für geradkettiges oder verzweigtes und gegebenenfalls durch Fluor und/oder Chlor substituiertes $C_1$-$C_4$-Alkandiyl steht,

X für Sauerstoff, Schwefel oder die Gruppierung N-$R^8$ steht worin,

$R^8$ für Wasserstoff oder gegebenenfalls durch Fluor und/oder Chlor, Cyano, $C_1$-$C_4$-Alkoxy, $C_1$-$C_4$-Alkoxy-carbonyl oder Pyridyl substituierte Reste aus der Reihe $C_1$-$C_4$-Alkyl, $C_3$-$C_4$-Alkenyl und $C_3$-$C_4$-Alkinyl steht und

Y für Sauerstoff oder Schwefel steht,

in welcher weiter

$R^4$ für Wasserstoff oder Halogen steht,

$R^5$ für Wasserstoff oder Halogen steht,

$R^6$ für Wasserstoff, Halogen oder gegebenenfalls durch Fluor und/oder Chlor substituiertes $C_1$-$C_4$-Alkyl steht,

$R^7$ für Wasserstoff, Halogen oder gegebenenfalls durch Fluor und/oder Chlor substituiertes $C_1$-$C_4$-Alkyl steht oder die beiden Reste

$R^6$ und $R^7$ zusammen für geradkettiges oder verzweigtes $C_3$-$C_5$-Alkandiyl stehen oder zusammen mit den Kohlenstoffatomen, an die sie gebunden sind, eine Benzogruppierung bilden, und

Q für Sauerstoff oder Schwefel steht.

**2.** Herbizide Mittel, gekennzeichnet durch einen Gehalt an mindestens einem N-Phenyl-substituierten Oxazindion der Formel (IA) gemäß Anspruch 1, in welcher

$R^1$ für Wasserstoff steht,

$R^2$ für Wasserstoff, Nitro, Cyano, Fluor, Chlor oder Brom oder für einen gegebenenfalls durch Fluor, Chlor, Cyclopropyl, welches durch Chlor und/oder Methyl substituiert sein kann; durch $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, Carboxy, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbony, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl, Di-($C_1$-$C_2$-alkyl)-aminocarbonyl, $C_1$-$C_2$-Alkoxy-$C_1$-$C_2$-alkyl-amino-carbonyl, $C_3$-$C_4$-Alkenyloxy-imino-$C_1$-$C_2$-alkyl, $C_1$-$C_2$-Alkoxy-imino-$C_1$-$C_2$-alkyl, $C_1$-$C_2$-Alkoxy-carbonyl-$C_1$-$C_2$-alkoxy-imino-$C_1$-$C_2$-alkyl, substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Cyano, Methyl oder Trifluormethyl steht, oder die beiden Reste

$R^2$ und $R^3$ zusammen für -O-$CH_2$-O-$CH_2$- oder die Gruppierung -X-$(CO)_n$-A-Y- stehen, worin

n für die Zahlen 0 oder 1 steht,

A für eine direkte Bindung oder für Methylen, Ethylen, Propylen oder Butylen steht,

X für Sauerstoff oder die Gruppierung N-$R^8$ steht, worin

| | |
|---|---|
| $R^8$ | für Wasserstoff, $C_1$-$C_4$-Alkyl, $C_3$-$C_4$-Alkenyl oder $C_3$-$C_4$-Alkinyl steht und |
| Y | für Sauerstoff steht, |

in welcher weiter

| | |
|---|---|
| $R^4$ | für Wasserstoff steht, |
| $R^5$ | für Wasserstoff, Fluor oder Chlor steht |
| $R^6$ | für Wasserstoff, Chlor, Brom, Methyl, Ethyl, Propyl, Isopropyl oder Trifluormethyl steht, |
| $R^7$ | für Wasserstoff, Chlor, Brom, Methyl, Ethyl, Propyl, Isopropyl oder Trifluormethyl steht oder die beiden Reste |
| $R^6$ und $R^7$ | zusammen für geradkettiges oder verzweigtes $C_3$-$C_5$-Alkandiyl stehen oder zusammen mit den Kohlenstoffatomen, an die sie gebunden sind, eine Benzogruppierung bilden, und |
| Q | für Sauerstoff oder Schwefel steht, |

**3.** Verfahren zur Bekämpfung von Unkräutern, dadurch gekennzeichnet, daß man N-Phenyl-substituierte Oxazindione der Formel (IA) gemäß den Ansprüchen 1 bis 2 auf die Unkräuter und/oder ihren Lebensraum einwirken läßt.

**4.** Verwendung von N-Phenyl-substituierten Oxazindionen der Formel (IA) gemäß den Ansprüchen 1 bis 2 zur Bekämpfung von Unkräutern.

**5.** Verfahren zur Herstellung von herbiziden Mitteln, dadurch gekennzeichnet, daß man N-Phenyl-substituierte Oxazindione der Formel (IA) gemäß den Ansprüchen 1 bis 2 mit Streckmitteln und/oder oberflächenaktiven Substanzen vermischt.

**6.** N-Phenyl-substituierte Oxazindione der allgemeinen Formel (I)

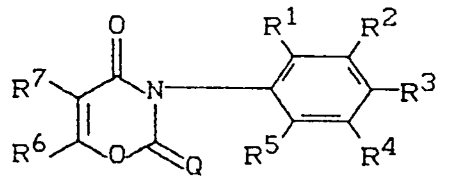

(I)

in welcher

$R^1$ bis $R^7$ und Q die in Anspruch 1 angegebene Bedeutung haben,

mit der Maßgabe, daß wenigstens drei der Reste $R^1$ bis $R^5$ von Wasserstoff verschieden sind oder die beiden Reste $R^2$ und $R^3$ zusammen für $-O-CH_2-O-CH_2-$ oder die Gruppierung $-X-(CO)_n-A-Y-$ stehen.

**7.** Verfahren zur Herstellung von N-Phenyl-substituierten Oxazindionen der allgemeinen Formel (I),

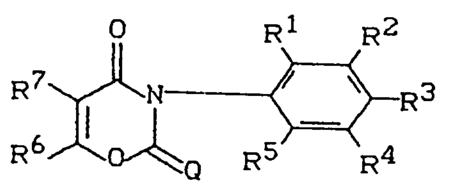

(I)

in welcher

$R^1$ bis $R^7$ und Q die in Anspruch 6 angegebene Bedeutung haben,

dadurch gekennzeichnet, daß man Aryliso(thio)cyanate der allgemeinen Formel (II)

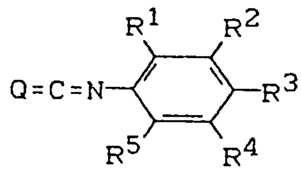

(II)

in welcher

Q, $R^1$, $R^2$, $R^3$, $R^4$ und $R^5$ die oben angegebenen Bedeutungen haben,

(a) mit 1,3-Dioxin-4-onen der allgemeinen Formel (III)

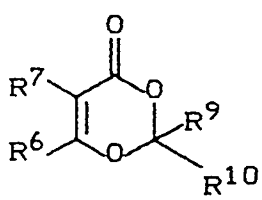

(III)

in welcher

$R^6$ und $R^7$ die oben angegebenen Bedeutungen haben und

$R^9$ und $R^{10}$ für Wasserstoff oder gegebenenfalls substituiertes Alkyl stehen,

gegebenenfalls in Gegenwart eines Verdünnungsmittels umsetzt, oder

(b) mit Dicarbonsäuredichloriden der allgemeinen Formel (IV)

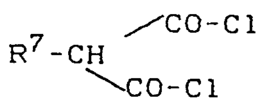

(IV)

in welcher

$R^7$ die oben angegebene Bedeutung hat,

gegebenenfalls in Gegenwart eines Verdünnungsmittels umsetzt, oder

(c) mit 2-Diazo-1,3-diketonen der allgemeinen Formel (V)

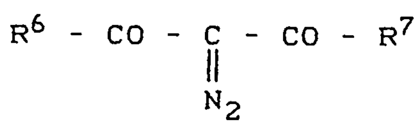

(V)

in welcher

$R^6$ und $R^7$ die oben angegebenen Bedeutungen haben,

gegebenenfalls in Gegenwart eines Verdünnungsmittels umsetzt, oder

(d) mit Salicylsäure oder deren Estern der allgemeinen Formel (VI)

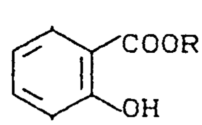

(VI)

in welcher

R für Wasserstoff oder Alkyl steht,

gegebenenfalls in Gegenwart eines Säureakzeptors und gegebenenfalls in Gegenwart eines Verdünnungsmittels umsetzt.

## Claims

**1.** Herbicidal agents, characterized in that they contain at least one N-phenyl-substituted oxazinedione of the formula (IA)

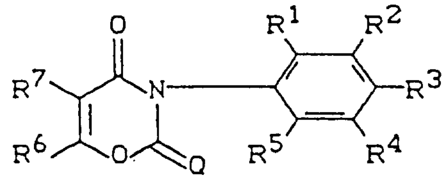

(IA)

in which

| | |
|---|---|
| $R^1$ | represents hydrogen or halogen, |
| $R^2$ | represents hydrogen, nitro, cyano, halogen, or represents a radical from the series comprising $C_1$-$C_8$-alkyl, $C_1$-$C_6$-alkoxy, $C_1$-$C_6$-alkylthio and $C_1$-$C_6$-alkylamino, which radical is optionally substituted by fluorine, chlorine, bromine, cyano, $C_3$-$C_6$-cycloalkyl which is optionally substituted by fluorine, chlorine, bromine, methyl and/or ethyl, by $C_1$-$C_6$-alkoxy, $C_1$-$C_4$-alkoxy-$C_1$-$C_4$-alkoxy, $C_1$-$C_6$-alkylthio, $C_1$-$C_6$-alkylsulphinyl, $C_1$-$C_6$-alkylsulphonyl, $C_1$-$C_4$-alkylthio-$C_1$-$C_4$-alkoxy, $C_1$-$C_4$-alkylsulphonyl-$C_1$-$C_4$-alkoxy, $C_3$-$C_6$-alkenyloxy, $C_3$-$C_6$-alkinyloxy, $C_3$-$C_6$-alkenylthio, $C_3$-$C_6$-alkinylthio, carboxyl, $C_1$-$C_6$-alkoxy-carbonyl, $C_3$-$C_6$-alkenyloxy-carbonyl, $C_3$-$C_6$-alkinyloxy-carbonyl, $C_3$-$C_6$-cycloalkyloxy-carbonyl, $C_3$-$C_6$-cycloalkenyloxy-carbonyl, $C_3$-$C_6$-cycloalkyl-$C_1$-$C_4$-alkoxy-carbonyl, $C_3$-$C_6$-cycloalkenyl-$C_1$-$C_4$-alkoxy-carbonyl, $C_1$-$C_4$-alkoxy-$C_1$-$C_4$-alkoxy-carbonyl, $C_1$-$C_4$-alkylthio-$C_1$-$C_4$-alkoxy-carbonyl, benzyloxy-$C_1$-$C_4$-alkoxy-carbonyl, $C_1$-$C_4$-alkoxy-carbonyl-$C_1$-$C_4$-alkoxy-carbonyl, $C_1$-$C_4$-alkylamino-carbonyl-$C_1$-$C_4$-alkoxy-carbonyl, di-($C_1$-$C_4$-alkyl)-amino-carbonyl-$C_1$-$C_4$-alkoxy-carbonyl, $C_1$-$C_4$-alkoxy-$C_1$-$C_4$-alkyl-amino-carbonyl, $C_1$-$C_4$-alkoxy-imino-$C_1$-$C_4$-alkyl, $C_3$-$C_4$-alkenyloxy-imino-$C_1$-$C_4$-alkyl, $C_1$-$C_4$-alkoxy-carbonyl-$C_1$-$C_4$-alkoxy-imino-$C_1$-$C_4$-alkyl, $C_1$-$C_6$-alkylthio-carbonyl, $C_3$-$C_6$-alkenylthio-carbonyl, $C_3$-$C_6$-alkinylthio-carbonyl, carbamoyl, $C_1$-$C_6$-alkylamino-carbonyl, $C_3$-$C_6$-alkenylamino-carbonyl, $C_3$-$C_6$-alkinylamino-carbonyl, di-($C_1$-$C_4$-alkyl)-amino-carbonyl, di-($C_3$-$C_4$-alkenyl)-amino-carbonyl and/or di-($C_3$-$C_4$-alkinyl)-amino-carbonyl, or represents a radical from the series comprising $C_3$-$C_6$-alkenyl, $C_3$-$C_6$-alkinyl, $C_3$-$C_6$-alkenyloxy, $C_3$-$C_6$-alkinyloxy, $C_3$-$C_6$-alkenylthio, $C_3$-$C_6$-alkinylthio, $C_1$-$C_6$-alkylsulphinyl and $C_1$-$C_6$-alkylsulphonyl, which radical is optionally substituted by fluorine and/or chlorine, |
| $R^3$ | represents hydrogen, halogen, nitro, cyano, or represents a radical from the series comprising $C_1$-$C_4$-alkyl, $C_3$-$C_4$-alkenyl, $C_3$-$C_4$-alkinyl, $C_1$-$C_4$-alkoxy, $C_3$-$C_4$-alkenyloxy, $C_3$-$C_4$-alkinyloxy, $C_1$-$C_4$-alkylthio, $C_3$-$C_4$-alkenylthio, $C_3$-$C_4$-alkinylthio, $C_1$-$C_4$-alkylsulphinyl, $C_1$-$C_4$-alkylsulphonyl and $C_1$-$C_4$-alkylamino, which radical is optionally substituted by fluorine and/or chlorine, or the two radicals |
| $R^2$ and $R^3$ | together represent -O-$CH_2$-O-$CH_2$- or the group -X-$(CO)_n$-A-Y- where |
| n | represents the numbers 0 or 1, |
| A | represents a direct bond or represents straight-chain or branched $C_1$-$C_4$-alkanediyl which is optionally substituted by fluorine and/or chlorine, |
| X | represents oxygen, sulphur or the group N-$R^8$ where |
| $R^8$ | represents hydrogen or radicals from the series comprising $C_1$-$C_4$-alkyl, $C_3$-$C_4$-alkenyl and $C_3$-$C_4$-alkinyl, which radicals are optionally substituted by fluorine and/or chlorine, cyano, $C_1$-$C_4$-alkoxy, $C_1$-$C_4$-alkoxy-carbonyl or pyridiyl, and |
| Y | represents oxygen or sulphur, |

in which furthermore

| | |
|---|---|
| $R^4$ | represents hydrogen or halogen, |
| $R^5$ | represents hydrogen or halogen, |
| $R^6$ | represents hydrogen, halogen or $C_1$-$C_4$-alkyl which is optionally substituted by fluorine and/or chlorine, |
| $R^7$ | represents hydrogen, halogen or $C_1$-$C_4$-alkyl which is optionally substituted by fluorine and/or chlorine, or the two radicals |

$R^6$ and $R^7$ together represent straight-chain or branched $C_3$-$C_5$-alkanediyl or together with the carbon atoms to which they are bonded form a benzo group, and

Q represents oxygen or sulphur.

**2.** Herbicidal agents, characterized in that they contain at least one N-phenyl-substituted oxazinedione of the formula (IA) according to Claim 1, in which

$R^1$ represents hydrogen,

$R^2$ represents hydrogen, nitro, cyano, fluorine, chlorine or bromine, or represents a radical from the series comprising $C_1$-$C_4$-alkyl, $C_1$-$C_4$-alkoxy, $C_1$-$C_4$-alkylthio and $C_1$-$C_4$-alkylamino, which radical is optionally substituted by fluorine, chlorine, cyclopropyl which can be substituted by chlorine and/or methyl; by $C_1$-$C_4$-alkoxy,$C_1$-$C_4$-alkylthio, $C_1$-$C_4$-alkylsulphinyl, $C_1$-$C_4$-alkylsulphonyl, $C_3$-$C_4$-alkenyloxy, $C_3$-$C_4$-alkinyloxy, $C_3$-$C_4$-alkenylthio, $C_3$-$C_4$-alkinylthio, carboxyl, $C_1$-$C_4$-alkoxy-carbonyl, $C_3$-$C_4$-alkenyloxy-carbonyl, $C_3$-$C_4$-alkinyloxy-carbonyl, $C_3$-$C_6$-cycloalkyloxy-carbonyl, $C_3$-$C_6$-cycloalkyl-$C_1$-$C_2$-alkoxy-carbonyl,$C_1$-$C_4$-alkoxy-$C_1$-$C_2$-alkoxy-carbonyl, $C_1$-$C_4$-alkylthio-$C_1$-$C_2$-alkoxy-carbonyl, $C_1$-$C_4$-alkoxy-carbonyl-$C_1$-$C_2$-alkoxy-carbonyl or $C_1$-$C_4$-alkylamino-carbonyl, di-($C_1$-$C_2$-alkyl)-aminocarbonyl, $C_1$-$C_2$-alkoxy-$C_1$-$C_2$-alkyl-amino-carbonyl, $C_3$-$C_4$-alkenyloxy-imino-$C_1$-$C_2$-alkyl, $C_1$-$C_2$-alkoxy-imino-$C_1$-$C_2$-alkyl, $C_1$-$C_2$-alkoxy-carbonyl-$C_1$-$C_2$-alkoxy-imino-$C_1$-$C_2$-alkyl, or represents a radical from the series comprising $C_3$-$C_4$-alkenyloxy, $C_3$-$C_4$-alkinyloxy, $C_3$-$C_4$-alkenylthio and $C_3$-$C_4$-alkinylthio, which radical is optionally substituted by fluorine and/or chlorine,

$R^3$ represents hydrogen, fluorine, chlorine, bromine, cyano, methyl or trifluoromethyl, or the two radicals

$R^2$ and $R^3$ together represent -O-$CH_2$-O-$CH_2$- or the group -X-$(CO)_n$-A-Y- where

n represents the numbers 0 or 1,

A represents a direct bond or represents methylene, ethylene, propylene or butylene,

X represents oxygen or the group N-$R^8$ where

$R^8$ represents hydrogen, $C_1$-$C_4$-alkyl, $C_3$-$C_4$-alkenyl or $C_3$-$C_4$-alkinyl, and

Y represents oxygen,

in which furthermore

$R^4$ represents hydrogen,

$R^5$ represents hydrogen, fluorine or chlorine,

$R^6$ represents hydrogen, chlorine, bromine, methyl, ethyl, propyl, isopropyl or trifluoromethyl,

$R^7$ represents hydrogen, chlorine, bromine, methyl, ethyl, propyl, isopropyl or trifluoromethyl, or the two radicals

$R^6$ and $R^7$ together represent straight-chain or branched $C_3$-$C_5$-alkanediyl or together with the carbon atoms to which they are bonded form a benzo group, and

Q represents oxygen or sulphur.

**3.** Method of combating weeds, characterized in that N-phenyl-substituted oxazinediones of the formula (IA) according to Claims 1 to 2 are allowed to act on the weeds and/or their environment.

**4.** Use of N-phenyl-substituted oxazinediones of the formula (IA) according to Claims 1 to 2 for combating weeds.

**5.** Process for the preparation of herbicidal agents, characterized in that N-phenyl-substituted oxazinediones of the formula (IA) according to Claims 1 to 2 are mixed with extenders and/or surface-active substances.

**6.** N-Phenyl-substituted oxazinediones of the general formula (I)

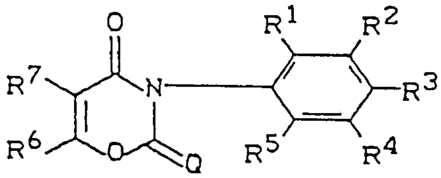

(I)

in which

$R^1$ to $R^7$ and Q have the meaning given in Claim 1,

with the proviso that at least three of the radicals $R^1$ to $R^5$ are other than hydrogen or the two radicals $R^2$ and $R^3$ together represent $-O-CH_2-O-CH_2-$ or the group $-X-(CO)_n-A-Y-$.

**7.** Process for the preparation of N-phenyl-substituted oxazinediones of the general formula (I)

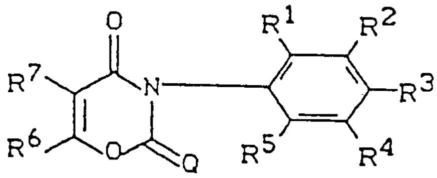

(I)

in which

$R^1$ to $R^7$ and Q have the meaning given in Claim 6,

characterized in that aryl iso(thio)cyanates of the general formula (II)

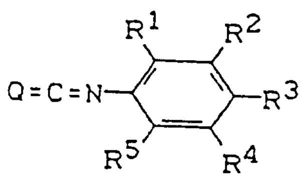

(II)

in which

Q, $R^1$, $R^2$, $R^3$, $R^4$ and $R^5$ have the abovementioned meanings,

(a) are reacted with 1,3-dioxin-4-ones of the general formula (III)

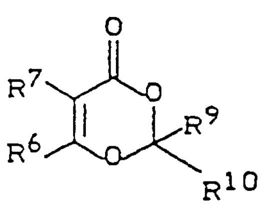

(III)

in which

$R^6$ and $R^7$ have the abovementioned meanings and

$R^9$ and $R^{10}$ represent hydrogen or optionally substituted alkyl,

if appropriate in the presence of a diluent, or

(b) are reacted with dicarboxylic acid dichlorides of the general formula (IV)

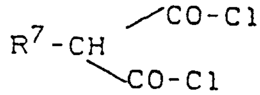

(IV)

in which

$R^7$ has the abovementioned meaning,

if appropriate in the presence of a diluent, or

(c) are reacted with 2-diazo-1,3-diketones of the general formula (V)

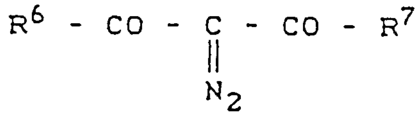

(V)

in which

$R^6$ and $R^7$ have the abovementioned meanings,

if appropriate in the presence of a diluent, or

(d) are reacted with a salicylic acid or its esters of the general formula (VI)

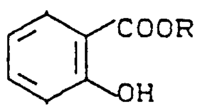

(VI)

in which

R represents hydrogen or alkyl,

if appropriate in the presence of an acid acceptor and if appropriate in the presence of a diluent.

## Revendications

**1.** Compositions herbicides, caractérisées par une teneur en au moins une oxazine-dione portant un substituant phényle sur l'azote, de formule (IA)

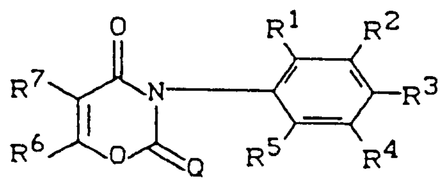

(IA)

dans laquelle

$R^1$ représente l'hydrogène ou un halogène,

$R^2$ est l'hydrogène, un groupe nitro, cyano, un halogène ou représente un reste de la série alkyle en $C_1$ à $C_8$, alkoxy en $C_1$ à $C_6$, alkylthio en $C_1$ à $C_6$ et alkylamino en $C_1$ à $C_6$ portant éventuellement un substituant fluor, chlore, brome, cyano, cycloalkyle en $C_3$ à $C_6$ éventuellement substitué par du fluor, du chlore, du brome, un radical méthyle et/ou éthyle, un substituant alkoxy en $C_1$ à $C_6$, (alkoxy en $C_1$ à $C_4$)-alkoxy en $C_1$ à $C_4$, alkylthio en $C_1$ à $C_6$, alkylsulfinyle en $C_1$ à $C_6$, alkylsulfonyle en $C_1$ à $C_6$, (alkylthio en $C_1$ à $C_4$)-alkoxy en $C_1$ à $C_4$, (alkylsulfonyle en $C_1$ à $C_4$)-alkoxy en $C_1$ à $C_4$, alcényloxy en $C_3$ à $C_6$, alcynyloxy en $C_3$ à $C_6$, alcénylthio en $C_3$ à $C_6$, alcynylthio en $C_3$ à $C_6$, carboxy, (alkoxy en $C_1$ à $C_6$)carbonyle, (alcényloxy en $C_3$ à $C_6$)carbonyle, (alcynyloxy en $C_3$ à $C_6$)carbonyle, (cycloalkyloxy en $C_3$ à $C_6$)-carbonyle, (cycloalcényloxy en $C_3$ à

$C_6$)carbonyle, (cycloalkyle en $C_3$ à $C_6$)-(alkoxy en $C_1$ à $C_4$)-carbonyle, (cycloalcényle en $C_3$ à $C_6$)-(alkoxy en $C_1$ à $C_4$)carbonyle, (alkoxy en $C_1$ à $C_4$)-(alkoxy en $C_1$ à $C_4$)-carbonyle, (alkylthio en $C_1$ à $C_4$)-(alkoxy en $C_1$ à $C_4$)carbonyle, benzyloxy-(alkoxy en $C_1$ à $C_4$)carbonyle, (alkoxy en $C_1$ à $C_4$)-carbonyl-(alkoxy en $C_1$ à $C_4$)carbonyle, (alkylamino en $C_1$ à $C_4$)carbonyl-(alkoxy en $C_1$ à $C_4$)-carbonyle, di(alkyle en $C_1$ à $C_4$)amino-carbonyl-(alkoxy en $C_1$ à $C_4$)-carbonyle, (alkoxy en $C_1$ à $C_4$)-(alkyle en $C_1$ à $C_4$)-aminocarbonyle, (alkoxy en $C_1$ à $C_4$)imino-(alkyle en $C_1$ à $C_4$), (alcényloxy en $C_3$ ou $C_4$)-imino-(alkyle en $C_1$ à $C_4$), alkoxy en $C_1$ à $C_4$)carbonyl-(alkoxy en $C_1$ à $C_4$)-imino-(alkyle en $C_1$ à $C_4$), (alkylthio en $C_1$ à $C_6$)carbonyle, (alcénylthio en $C_3$ à $C_6$)carbonyle, (alcynylthio en $C_3$ à $C_6$)carbonyle, carbamoyle, (alkylamino en $C_1$ à $C_6$)carbonyle, (alcénylamino en $C_3$ à $C_6$)carbonyle, (alcynylamino en $C_3$ à $C_6$)carbonyle, di(alkyle en $C_1$ à $C_4$)aminocarbonyle, di(alcényle en $C_3$ ou $C_4$)aminocarbonyle et/ou di(alcynyle en $C_3$ ou $C_4$)aminocarbonyle ou un reste de la série alcényle en $C_3$ à $C_6$, alcynyle en $C_3$ à $C_6$, alcényloxy en $C_3$ à $C_6$, alcynyloxy en $C_3$ à $C_6$, alcénylthio en $C_3$ à $C_6$, alcynylthio en $C_3$ à $C_6$, alkylsulfinyle en $C_1$ à $C_6$ et alkylsulfonyle en $C_1$ à $C_6$ substitués le cas échéant par du fluor et/ou du chlore,

$R^3$ représente l'hydrogène, un halogène, un groupe nitro, cyano ou un reste de la série alkyle en $C_1$ à $C_4$, alcényle en $C_3$ ou $C_4$, alcynyle en $C_3$ ou $C_4$, alkoxy en $C_1$ à $C_4$, alcényloxy en $C_3$ ou $C_4$, alcynyloxy en $C_3$ ou $C_4$, alkylthio en $C_1$ à $C_4$, alcénylthio en $C_3$ ou $C_4$, alcynylthio en $C_3$ ou $C_4$, alkylsulfinyle en $C_1$ à $C_4$, alkylsulfonyle en $C_1$ à $C_4$ et alkylamino en $C_1$ à $C_4$ portant éventuellement un substituant fluor et/ou chlore, ou bien les deux restes

$R^2$ et $R^3$ forment conjointement un groupe $-O-CH_2-O-CH_2-$ ou le groupement $-X-(CO)_n-A-Y-$ dans lequel

n représente les nombres 0 ou 1,

A est une liaison directe ou représente un groupe alcanediyle en $C_1$ à $C_4$ à chaîne droite ou ramifiée et éventuellement substitué par du fluor et/ou du chlore,

X est l'oxygène, le soufre ou le groupement $N-R^8$ dans lequel

$R^8$ représente l'hydrogène ou des restes de la série alkyle en $C_1$ à $C_4$, alcényle en $C_3$ ou $C_4$ et alcynyle en $C_3$ ou $C_4$ portant éventuellement un substituant fluoro et/ou chloro, cyano, alkoxy en $C_1$ à $C_4$, (alkoxy en $C_1$ à $C_4$)carbonyle ou pyridyle et

Y représente l'oxygène ou le soufre,

dans laquelle en outre

$R^4$ représente l'hydrogène ou un halogène,

$R^5$ représente l'hydrogène ou un halogène,

$R^6$ est l'hydrogène, un halogène ou un reste alkyle en $C_1$ à $C_4$ éventuellement substitué par du fluor et/ou du chlore,

$R^7$ est l'hydrogène, un halogène ou un groupe alkyle en $C_1$ à $C_4$ éventuellement substitué par du fluor et/ou du chlore ou bien les deux restes

$R^6$ et $R^7$ représentent un groupe alcanediyle en $C_3$ à $C_5$ à chaîne droite ou ramifiée ou forment, conjointement avec les atomes de carbone auxquels ils sont lies, un groupement benzo et

Q est l'oxygène ou le soufre.

**2.** Compositions herbicides, caractérisées par une teneur en au moins une oxazinedione à substituant phényle sur l'azote, de formule (IA) suivant la revendication 1, dans laquelle

$R^1$ représente l'hydrogène,

$R^2$ représente l'hydrogène, un groupe nitro, cyano, du fluor, du chlore ou du brome ou un reste de la série alkyle en $C_1$ à $C_4$, alkoxy en $C_1$ à $C_4$, alkylthio en $C_1$ à $C_4$ ou alkylamino en $C_1$ à $C_4$, éventuellement substitué par du fluor, du chlore, un groupe cyclopropyle qui peut porter éventuellement un substituant chloro et/ou méthyle ; par un groupe alkoxy en $C_1$ à $C_4$, alkylthio en $C_1$ à $C_4$, alkylsulfinyle en $C_1$ à $C_4$, alkylsulfonyle en $C_1$ à $C_4$, alcényloxy en $C_3$ ou $C_4$, alcynyloxy en $C_3$ ou $C_4$, alcénylthio en $C_3$ ou $C_4$, alcynylthio en $C_3$ ou $C_4$, carboxy, (alkoxy en $C_1$ à $C_4$)carbonyle, (alcényloxy en $C_3$ ou $C_4$)-carbonyle, (alcynyloxy en $C_3$ ou $C_4$)carbonyle, (cycloalkyloxy en $C_3$ à $C_6$)carbonyle, (cycloalkyle en $C_3$ à $C_6$)-(alkoxy en $C_1$ ou $C_2$)-carbonyle, (alkoxy en $C_1$ à $C_4$)-(alkoxy en $C_1$ ou $C_2$)-carbonyle, (alkylthio en $C_1$ à $C_4$)-(alkoxy en $C_1$ ou $C_2$)carbonyle, (alkoxy en $C_1$ à $C_4$)carbonyl-(alkoxy en $C_1$ ou $C_2$)carbonyle ou un groupe (alkylamino en $C_1$ à $C_4$)-

carbonyle, di(alkyle en $C_1$ ou $C_2$)aminocarbonyle, (alkoxy en $C_1$ ou $C_2$)-alkyle en $C_1$ ou $C_2$)aminocarbonyle, alcényloxy en $C_3$ ou $C_4$)imino-(alkyle en $C_1$ ou $C_2$), (alkoxy en $C_1$ ou $C_2$)imino-(alkyle en $C_1$ ou $C_2$), (alkoxy en $C_1$ ou $C_2$)carbonyl-(alkoxy en $C_1$ ou $C_2$)-imino-(alkyle en $C_1$ ou $C_2$) ou un reste de la série alcényloxy en $C_3$ ou $C_4$, (alcynyloxy en $C_3$ ou $C_4$), alcénylthio en $C_3$ ou $C_4$ et alcynylthio en $C_3$ ou $C_4$ éventuellement substitué par du fluor et/ou du chlore,

$R^3$ représente l'hydrogène, le fluor, le chlore, le brome, un groupe cyano, méthyle ou trifluorométhyle, ou bien les deux restes

$R^2$ et $R^3$ forment conjointement un groupe $-O-CH_2-O-CH_2-$ ou le groupement $-X-(CO)_n-A-Y-$, dans lequel

n représente les nombres 0 ou 1,

A est une liaison directe ou représente un groupe méthylène, éthylène, propylène ou butylène,

X est l'oxygène ou le groupement $N-R^8$, dans lequel

$R^8$ est l'hydrogène, un groupe alkyle en $C_1$ à $C_4$, alcényle en $C_3$ ou $C_4$ ou alcynyle en $C_3$ ou $C_4$ et

Y représente l'oxygène,

dans lequel en outre

$R^4$ représente l'hydrogène,

$R^5$ est l'hydrogène, le fluor ou le chlore,

$R^6$ représente l'hydrogène, le chlore, le brome, un groupe méthyle, éthyle, propyle, isopropyle ou trifluorométhyle,

$R^7$ représente l'hydrogène, le chlore, le brome, un groupe méthyle, éthyle, propyle, isopropyle ou trifluorométhyle ou bien les deux restes

$R^6$ et $R^7$ forment ensemble un groupe alcanediyle en $C_3$ à $C_5$ à chaîne droite ou ramifiée ou forment conjointement avec les atomes de carbone auxquels ils sont liés un groupement benzo et

Q représente l'oxygène ou le soufre.

**3.** Procédé pour combattre des mauvaises herbes, caractérisé en ce qu'on fait agir sur les mauvaises herbes et/ou sur leur milieu des oxazinediones portant un substituant phényle sur l'azote, de formule (IA) suivant les revendications 1 et 2.

**4.** Utilisation d'oxazinediones portant un substituant phényle sur l'azote, de formule (IA) suivant les revendications 1 ou 2 pour combattre des mauvaises herbes.

**5.** Procédé de préparation de compositions herbicides, caractérisé en ce qu'on mélange des oxazinediones à substituant phényle sur l'azote de formule (IA) suivant les revendications 1 et 2 avec des diluants et/ou des agents tensio-actifs.

**6.** Oxazinediones à substituant phényle sur L'azote, de formule générale (I)

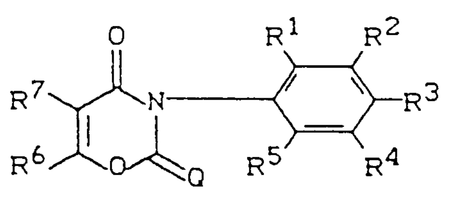

(I)

dans laquelle

$R^1$ à $R^7$ et Q ont les définitions indiquées dans la revendication 1,

sous réserve qu'au moins trois des restes $R^1$ à $R^5$ soient différents de l'hydrogène ou que les deux restes $R^2$ et $R^3$ forment ensemble un groupe $-O-CH_2-O-CH_2-$ ou le groupement $-X-(CO)_n-A-Y-$.

**7.** Procédé de production d'oxazinediones à substituant N-phényle sur l'azote de formule générale (I),

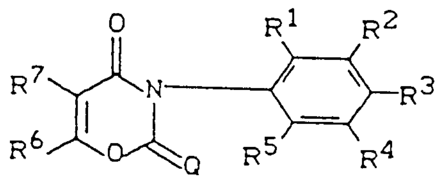

(I)

dans laquelle
$R^1$ à $R^7$ et Q ont les définitions indiquées dans la revendication 6,
caractérisé en ce qu'on fait réagir des aryliso(thio)cyanates de formule générale (II)

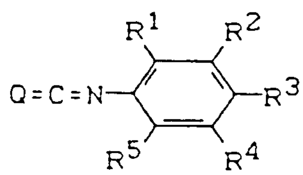

(II)

dans laquelle
Q, $R^1$, $R^2$, $R^3$, $R^4$ et $R^5$ ont les définitions indiquées ci-dessus,
(a) avec des 1,3-dioxine-4-ones de formule générale (III)

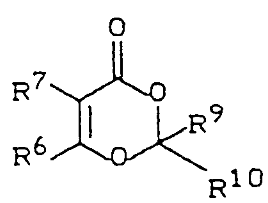

(III)

dans laquelle
$R^6$ et $R^7$ ont les définitions indiquées ci-dessus et
$R^9$ et $R^{10}$ représentent l'hydrogène ou un groupe alkyle éventuellement substitué,
le cas échéant en présence d'un diluant, ou bien
(b) avec des dichlorures d'acides dicarboxyliques de formule générale (IV)

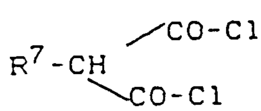

(IV)

dans laquelle
$R^7$ a la définition indiquée ci-dessus,
le cas échéant en présence d'un diluant, ou bien
(c) avec des 2-diazo-1,3-dicétones de formule générale (V)

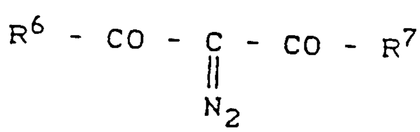

(V)

dans laquelle

$R^6$ et $R^7$ ont les définitions indiquées ci-dessus,
le cas échéant en présence d'un diluant, ou bien
(d) avec l'acide salicylique ou ses esters de formule générale (VI)

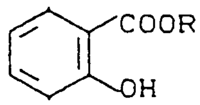

(VI)

dans laquelle
R représente l'hydrogène ou un groupe alkyle,
le cas échéant en présence d'un accepteur d'acide et en la présence éventuelle d'un diluant.